(19) Europäisches Patentamt / European Patent Office / Office européen des brevets

(11) **EP 4 452 233 B1**

(12) **EUROPEAN PATENT SPECIFICATION**

(45) Date of publication and mention
of the grant of the patent:
**24.12.2025 Bulletin 2025/52**

(21) Application number: **22843886.7**

(22) Date of filing: **20.12.2022**

(51) International Patent Classification (IPC):
*A61K 9/16* (2006.01)   *A61K 9/19* (2006.01)
*A61K 38/39* (2006.01)   *A61K 47/18* (2017.01)
*A61K 31/198* (2006.01)   *A61K 38/17* (2006.01)
*A61K 38/37* (2006.01)   *A61K 38/45* (2006.01)
*A61K 38/36* (2006.01)

(52) Cooperative Patent Classification (CPC):
(C-Sets available)
A61K 9/0019; A61K 9/1617; A61K 9/1694;
A61K 9/19; A61K 31/198; A61K 38/1709;
A61K 38/363; A61K 38/37; A61K 38/39;
A61K 38/45; A61K 47/183          (Cont.)

(86) International application number:
**PCT/IL2022/051356**

(87) International publication number:
**WO 2023/119277 (29.06.2023 Gazette 2023/26)**

(54) **HIGHLY SOLUBLE FIBRINOGEN COMPOSITIONS**

HOCHLÖSLICHE FIBRINOGENZUSAMMENSETZUNGEN

COMPOSITIONS DE FIBRINOGÈNE HAUTEMENT SOLUBLES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **21.12.2021  IL 28922421**
         **21.12.2021  US 202163292162 P**

(43) Date of publication of application:
**30.10.2024  Bulletin 2024/44**

(73) Proprietor: **Omrix Biopharmaceuticals Ltd.**
**5510801 Kiyat Ono (IL)**

(72) Inventors:
 • **NUR, Israel**
  **7403769 Nes-Ziona (IL)**
 • **GRIMBERG, Elena**
  **7656626 Rehovot (IL)**
 • **PODOLER, Itai**
  **7630412 Rehovot (IL)**
 • **TZELIK, Shirly**
  **7529126 Rishon LeZion (IL)**

(74) Representative: **Carpmaels & Ransford LLP**
**One Southampton Row**
**London WC1B 5HA (GB)**

(56) References cited:
**WO-A1-93/22336       CN-A- 113 563 457**
**US-A1- 2010 203 033   US-B2- 7 727 743**

(52) Cooperative Patent Classification (CPC): (Cont.)

C-Sets
**A61K 31/198, A61K 2300/00;**
**A61K 38/1709, A61K 2300/00;**
**A61K 38/363, A61K 2300/00;**
**A61K 38/37, A61K 2300/00;**
**A61K 38/39, A61K 2300/00;**
**A61K 38/45, A61K 2300/00**

**Description**

FIELD OF THE INVENTION

The present invention relates, inter alia, to fibrinogen compositions for intravenous administration.

BACKGROUND OF THE INVENTION

Fibrinogen is a coagulation factor which can be converted to fibrin by an enzymatic reaction involving thrombin and factor XIII and promotes the formation of a hemostatic plug with activated platelets in case of bleeding. Coagulation disorders are common in severe acute hemorrhages (SAH) and are directly or indirectly related to excessive bleeding. Among plasma coagulation factors, fibrinogen level is the first to reach critical values in such cases. Acquired or congenital shortage of fibrinogen is often treated by intravenous (IV) administration of either cryoprecipitate or exogenous purified fibrinogen solution. Fibrinogen or plasma cryoprecipitate replacement therapy is currently indicated as prophylaxis and therapy of hemorrhage in congenital and acquired fibrinogen deficiency, this latter being associated with liver failure, disseminated intravascular coagulation, massive transfusion and cardiac surgery (Tziomalos K, et al., Vasc Health Risk Manag. 2009;5:843; Weinkove R, Rangarajan S. Fibrinogen Transfus Med. 2008;18:151-7).

A solid fibrinogen composition derived from viral inactivated cryo-precipitate of human plasma comprises mainly fibrinogen, and may also contain significant quantities of other proteins such as, e.g., fibronectin and FXIII, that have shown to enhance the effectiveness in restoring blood-coagulation ability in case of SAH and to enhances the restoration of a damaged tissue.

Hypofibrinogenemia is defined by a decreased level of normal fibrinogen between 0.5 g/L and the lower limit of the normal range for the local laboratory (usually 1.5 g/L). Fibrinogen supplementation can be provided by transfusion of fresh-frozen plasma (FFP), cryoprecipitate and fibrinogen concentrate (O'Shaughnessy DF, Atterbury C, Bolton Maggs P, et al; Br J Haematol. 2004;126:11-28; Sørensen B, Bevan D. Br J Haematol. 2010;149:834-43). However, as FFP has several limitations including a low fibrinogen content, which means that large volumes must be given, and the risk of transfusion-related complications (e.g., transfusion-related acute lung injury [TRALI] and viral transmission).

Arginine is a biologically active substance and can be administered intravenously to treat high blood pressure in the lungs or to stimulate the pituitary gland to produce growth hormone for the diagnosis of certain conditions (R-Gene 10).

Plasma cryoprecipitate is used for over 50 years either prophylactically or as a treatment for replacement of plasma protein due to severe bleeding in emergency situations. The long history of using plasma cryoprecipitate placed cryoprecipitate as the safest drug for replacement therapy for fibrinogen FXIII factor VIII. However, fibrinogen cryoprecipitate is among the least soluble plasma proteins. Thus, it is time consuming to dissolve a solid fibrinogen plasma cryoprecipitate preparation and obtain a solution with approximately 20 mg/ml fibrinogen without the use of aggressive mixing and/or vacuum sealing, otherwise reconstitution may result in foaming that might lead to possible protein degradation.

There are other drawbacks that limit the use of frozen plasma cryoprecipitate: the time it takes to infuse it to the patient, the cold (frozen) supply chain, and the risk of transmissible agent, e.g., viruses and prions. In addition, logistic issues, such as the short shelf-life and its availability in places far from a blood bank. These deficiencies lead to the proliferation product where plasma cryoprecipitate has been viral inactivated, concentrated, and lyophilized.

The quantitates of fibrinogen needed to replace the amounts lost during a sever exsanguination are significant. Recent studies (Massimo F. and Giuseppe L. Fibrinogen replacement therapy: a critical review of the literature, Blood Transfusion. 2012 Jan; 10(1): 23-27) suggest that the median single and total doses per episode should be 2.0 - 4.0 g per patient. Yet, it may take more than 20 min to fully reconstitute a vial containing a cryoprecipitate with 1 gr of fibrinogen. This calls for developing a massive infusion of fibrinogen in a short period.

International Application Publication No. WO1998/055140 discloses a fibrinogen concentrate wherein the concentration of fibrinogen is less than 80 % of the total protein concentration and other naturally occurring plasma proteins such as fibronectin, factor VIII, von Willebrand factor, factor XIII, vitronectin, are present in amounts of at least 20 % of the total protein concentration.

US Patent No. 6121232 discloses a stabilized solution of fibrinogen containing samples, in particular a stabilized solution of the biological active component (BAC) which is a solution of proteins derived from blood plasma comprising fibrinogen, tranexamic acid and arginine or lysine or mixtures or arginine and lysine, their pharmaceutically acceptable salts, as well as, optionally, substances forming a buffered solution in aqueous medium.

US Patent No. 7727743 relates to a method for obtaining cryoprecipitatable proteins, comprising a viral inactivation step by thermally treating a lyophilisate of these proteins, comprising, before rendering the proteins in the form of a lyophilisate, an initial addition step, to these proteins, of a stabilizing and solubilizing formulation containing a mixture consisting of arginine, at least one hydrophobic amino acid and of tribasic sodium citrate; and to a concentrate consisting of at least one cryoprecipitable protein containing the stabilizing and solubilizing formulation introduced according to the method and being suited for therapeutic use.

US Patent No. 9943600 relates to a method for stabilising a human blood protein or human blood plasma protein with a molecular weight of >10 KDa by adding melezitose to a solution comprising the human blood protein or human blood plasma protein with a molecular weight of >10 KDa.

US Patent No. 9814765 discloses a single component sealant formulation (e.g., in a liquid form), methods for its preparation, and use. The formulation includes fibrinogen; vitamin K-dependent clotting zymogens comprising at least Factor II (FII) and Factor X (FX).

US Patent Application No. 2013/0312868 discloses methods and devices for dissolving solid protein compositions, such as solid compositions comprising fibrinogen, in an aqueous solvent. The methods comprise use of a closed container containing a volume of solid fibrinogen composition and a head space wherein the pressure within the headspace is sub-atmospheric. Aqueous solvent is introduced into the container while maintaining the sub-atmospheric pressure, and subsequent to addition of the solvent, the size of the headspace is decreased to bring the pressure to atmospheric pressure.

There is an unmet need for a safe plasma cryoprecipitate comprising a concentrated, fibrinogen characterized by fast reconstitution that would allow for a consistent dosing at low volume with no blood type matching required.

## SUMMARY OF THE INVENTION

A solid fibrinogen product such as plasma cryoprecipitate characterized by a fast dissolution without significant foaming and protein degradation is much needed, as it will extend the product shelf life, simplify the supply chain and make accessible for treatment in emergency cases of severe acute hemorrhages (SAH).

An object of the present invention is to provide a formulation that would generate a solid fibrinogen composition such as homogenous lyophilization cake or a powder made therefrom that can be easily dissolved e.g., within less than 20 min at atmospheric conditions and room temperatures, and would fully preserve the proteins potency after the reconstitution, without generating undesired turbidity or foaming.

The inventors have found that, surprisingly, arginine can improve the solubility of a solid fibrinogen composition. Thus, when added to a formulation in certain quantities, arginine may significantly shorten the reconstitution time of a solid fibrinogen composition, for example, lyophilized cryoprecipitate preparation. It is noteworthy that arginine is a chaotropic agent (i.e. a compound which disrupts hydrogen bonding in aqueous solution, and changes the protein folding) and it might permanently denature large proteins such as fibrinogen and FVIII if used in high concentrations since it might prevent the renaturation of the protein and the restoration of its natural folding. Furthermore, arginine may be potentially over-dosed if administered intravenously in large amounts. Nonetheless, the inventors have found an unexpected concentration range of arginine which, on the one hand, facilitates fast dissolution of renatured (i.e. natural folding being restored) fibrinogen composition e.g., lyophilized cryoprecipitate, and, on the other hand, does not cause adverse effects or significant reduction of the proteins potency.

Each aspect and embodiment of the present disclosure presented below may be combined with any other aspect and embodiment unless specified otherwise. Claimed is a composition comprising fibrinogen, Factor VIII, and a positively charged amino acid, wherein the positively charged amino acid comprises arginine, wherein the positively charged amino acid is present at a concentration ranging from above $35 \times 10^{-3}$ mmol per cm$^3$ to below $142 \times 10^{-3}$ mmol per cm$^3$, and wherein the ratio of arginine to total protein ranges from above 0.2 mg per mg protein to below 1 mg of arginine per mg protein.

According to an aspect of some embodiments of the present invention, there is provided a composition comprising fibrinogen, Factor VIII, and a positively charged amino acid, wherein the ratio of the positively charged amino acid to Factor VIII ranges from above 1.4 to below 8.3 mg/IU, or from 6 to 47.5 mmol per IU.

According to another aspect of some embodiments of the present invention, there is provided a composition comprising fibrinogen, Factor VIII, and a positively charged amino acid, wherein the positively charged amino acid is present at a concentration ranging from above 7.5 mg/ml to below 30 mg/ml, or from 35 to 142 mM (which is $35 \times 10^{-3}$ mmol per cm$^3$ to $142 \times 10^{-3}$ mmol per cm$^3$).

According to the claims, the positively charged amino acid comprises arginine. In some embodiments, the arginine is present as and refers to a salt e.g., arginine hydrochloride.

In some embodiments of any aspect, the composition is formulated as a pharmaceutical composition for intravenous administration. In some embodiments of any aspect, the composition is substantially devoid of added hydrophobic amino acid.

In some embodiments of any aspect, the composition comprises about 5% to about 25% albumin, by weight of the total proteins.

In some embodiments of any aspect, the composition is viral-inactivated.

In some embodiments of any aspect, the composition is substantially devoid of added albumin.

In some embodiments of any aspect, the composition is in a solid form.

In some embodiments of any aspect, the composition is selected from sponge-like uniform form, or a powder.

In some embodiments of any aspect, the composition is in a solid form (e.g., in amorphic or the sponge-like uniform form, or in the powder form) and is characterized by dissolution in an aqueous medium within less than 20 min at an atmospheric pressure and 25 °C.

In some embodiments of any aspect, the composition is characterized by dissolution in an aqueous medium within less than 5 min.

In some embodiments of any aspect, the ratio of arginine to fibrinogen ranges from 0.3 to about than 1.6, respectively, by weight.

In some embodiments of any aspect, the composition further comprises one or more members selected from: factor XIII, fibronectin, and von Willebrand factor, and vitronectin.

According to the claims, the ratio of arginine to total protein ranges from above 0.2 mg per mg protein to below 1 mg of arginine per mg protein.

According to another aspect of some embodiments of the present invention, there is provided a dry pharmaceutical composition capable of dissolving within in less than 6 mins in an aqueous medium, the composition comprising Factor VIII, arginine and fibrinogen, wherein: (i) the ratio of arginine to Factor VIII ranges from above 1.4 to below 8.3 mg/IU, or from 6 to 47.5 mmol/IU; (ii) the ratio of arginine to total protein ranges from above 0.2 mg per mg protein to below 1 mg of arginine per mg protein, and (iii) wherein the ratio of arginine to fibrinogen ranges from 0.3 to about than 1.6, by weight.

According to another aspect of some embodiments of the present invention, there is provided a method for the preparation ("the preparation method") of a fibrinogen- and factor VIII-containing product in the solid form, the method comprising the step of drying a solution comprising fibrinogen, factor VIII, a positively charged amino acid, wherein the ratio of the positively charged amino acid to Factor VIII ranges from above 1.4 to below 8.3 mg/IU, or from 6 to 47.5 mmol/IU.

In the claims of the preparation method, the positively charged amino acid comprises arginine, and the ratio of arginine to total protein ranges from above 0.2 mg per mg protein to below 1 mg of arginine per mg protein. .

In some embodiments of the preparation method, the solution is viral inactivated.

In some embodiments of the preparation method, the step of drying is carried out by lyophilization.

In some embodiments of the preparation method, the method comprises at least two orthogonal viral inactivation steps of the solution.

According to another aspect of some embodiments of the present invention, there is provided a method for obtaining a high-soluble solid composition comprising fibrinogen and Factor VIII, the method comprising adding a positively charged amino acid to a liquid composition comprising the fibrinogen and Factor VIII in a ratio of the positively charged amino acid to Factor VIII ranging from above 1.4 to below 8.3 mg/IU, respectively (or from 6 to 47.5 mmol/IU); and drying the liquid composition so as to obtain the solid composition.

In the claims of the method for obtaining a highly soluble solid composition, the positively charged amino acid comprises arginine, and the ratio of arginine to total protein ranges from above 0.2 mg per mg protein to below 1 mg of arginine per mg protein.

In some embodiments of the method for obtaining a highly soluble solid composition, the method is devoid of adding a hydrophobic amino acid.

In some embodiments, there is provided a solid composition obtained by the method for obtaining a highly soluble solid composition. In some embodiments, the highly soluble solid composition is characterized by dissolving in an aqueous medium within less than 5 min.

In some embodiments, the composition obtained by the method for obtaining a highly soluble solid composition is characterized by a ratio of arginine to fibrinogen ranging from 0.3 to about than 1.6, respectively, by weight.

In some embodiments, the composition obtained by the method for obtaining a highly soluble solid composition comprises one or more members selected from: factor XIII, fibronectin, and von Willebrand factor, and vitronectin.

According to another aspect, there is provided a method for obtaining a reconstituted solid fibrinogen in an aqueous medium, the method comprising providing the disclosed solid composition, e.g., obtainable by lyophilization; and adding an aqueous medium at a volume ranging from above 100% to less than 170%, e.g., about 125%, of the solid fibrinogen. In some embodiments, there is provided a reconstituted solid fibrinogen in an aqueous medium obtainable by the method.

According to another aspect, there is provided a kit for obtaining a reconstituted solid fibrinogen, the kit comprising a first container comprising the disclosed solid composition, e.g., obtainable by lyophilization; and a second container comprising an aqueous medium, optionally being a sterile medium, optionally at a volume ranging from above 100% to less than 170%, e.g., about 125%, of the solid fibrinogen.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

BRIEF DESCRIPTION OF THE DRAWINGS

Some embodiments of the invention are herein described, by way of example only, with reference to the accompanying Figure. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

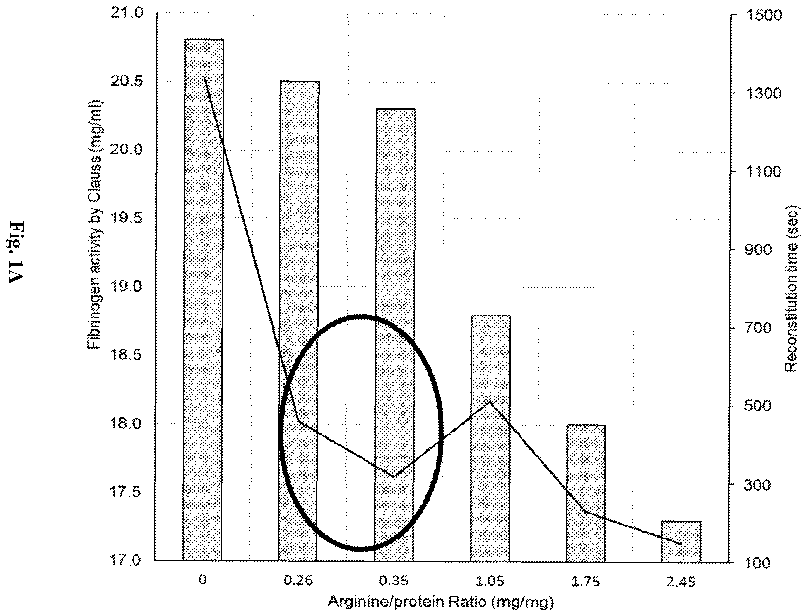

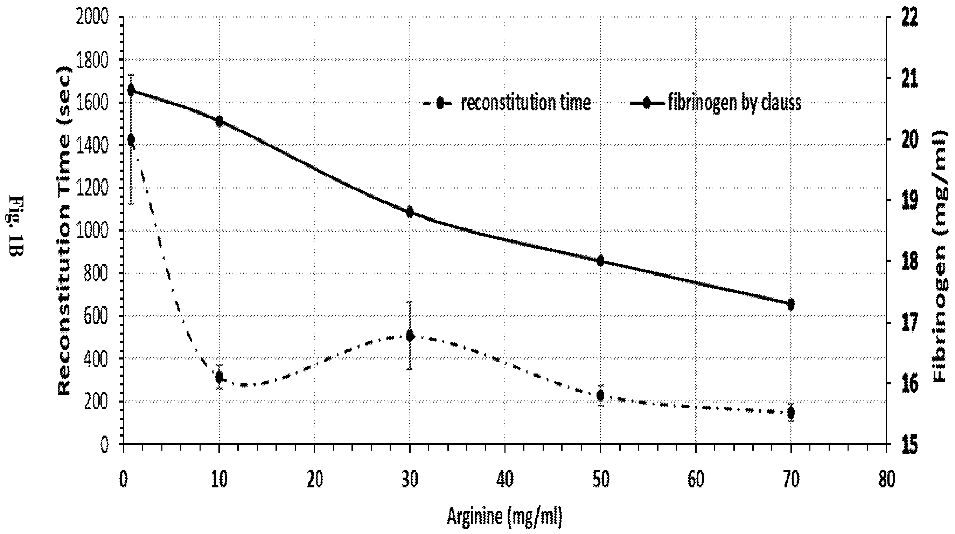

**Figures 1A-1B** present graphs summarizing the effect of the arginine-to-total protein ratio (Figure 1A; the bar graph relates to fibrinogen activity, line graph relates to the reconstitution time, the circle shows the optimal range), and of arginine concentration (Figure 1B; reconstitution time is presented as a polynomial regression) on the reconstitution time, recovery of fibrinogen and/or of Factor VIII in viral inactivate cryoprecipitate.

DESCRIPTION OF SOME EMBODIMENTS OF THE INVENTION

The present disclosure provides, in accordance with some aspects, a composition comprising Factor VIII, polar or positively charged ("basic") amino acid, and fibrinogen, with the ratio of the basic or polar amino acid to Factor VIII ranging from above 6 to below 47.5 mmol/IU. In another aspect of the present disclosure, there is provided a composition comprising Factor VIII, polar or basic amino acid, and fibrinogen, wherein the polar or basic amino acid e.g., arginine, is present at a concentration ranging from above 7.5 mg/ml to below 30 mg/ml. The claims demand the presence of arginine, wherein the ratio of arginine to total protein ranges from above 0.2 mg per mg protein to below 1 mg of arginine per mg protein.
In some embodiments of any aspect, the composition is for lyophilization. In some embodiments of any aspect, the composition is or has been lyophilized.
The term "amino acid" or "amino acids" is understood to include the 20 naturally occurring amino acids; those amino acids often modified post-translationally in vivo, including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids.
The term "polar amino acid" refers to a hydrophilic amino acid, typically but not exclusively, having a side chain that is uncharged at physiological pH, and which has at least one bond in which the pair of electrons shared in common by two atoms is held more closely by one of the atoms. Typically, polar amino acids possess amide groups in their side chains which usually generate or interfere with hydrogen-bonded inside the protein, giving rise to a change in the corresponding protein folding. Exemplary polar amino acids include arginine, asparagine, glutamine, or histidine.
The term "positively charged amino acid" (also referred to herein as "basic amino acid") as used herein refers to an amino acid residue or side chain that is or is capable of being positively charged at physiological pH (about 7.4 in plasma).
In some embodiments of any aspect, the positively charged acid is selected from, without being limited thereto, arginine, histidine, lysine. The claims demand that the positively charged amino acid comprises arginine. In exemplary embodiments, the polar amino acid, which is also positively charged amino acid, is arginine.
As used herein, the term "IU" denotes "International Units" and may be determined by the clotting assay against an internal reference standard for potency concentration measurement that has been calibrated against, for example, the relevant World Health Organization (WHO) Second International Standard. A unit (U) is equivalent to an International Unit (IU).
The term "Factor VIII" as used herein refers to coagulation Factor VIII or mimetic(s) thereof. Factor VIII may be selected from human Factor VIII, recombinant Factor VIII and porcine factor VIII.
As used herein and in the art, the term "fibrinogen" refers to a precursor protein of the blood clot matrix. The fibrinogen has a molecular weight of about 340,000 Daltons and consists of 3 pairs of non-identical polypeptide chains, A$\alpha$, B$\beta$ and $\gamma$, linked together by disulfide bonds. Typically, fibrinogen has a trinodular structure: two identical D terminal globular domains and a central E globular domain connected by supercoiled $\alpha$-helices.
Accordingly, in some embodiments, the solid compositions disclosed herein comprise fibrinogen in a solid state. In some embodiments, the solid fibrinogen compositions described herein comprise fibrinogen as the main ingredient, and further include other ingredients e.g., other proteins, including e.g., arginine. The fibrinogen in the composition can be blood-derived or recombinant. Examples of proteins other than fibrinogen present in the composition include, but are not limited to, fibronectin, factor VIII, von Willebrand factor, and factor XIII. In one embodiment of any aspect of the invention, the composition originates from a cryoprecipitate. In one embodiment of the invention, plasminogen is specifically removed or depleted from the cryoprecipitate in order to delay or stop the fibrinolysis (as described in U.S. Pat. Nos. 5,792,835 and 7,125,569).

The fibrinogen may be purified and isolated from plasma. Fibrinogen containing component can be a biologically active component (BAC) such as a fibrinogen concentrated viral-inactivated cryoprecipitate from human plasma comprising solution. BAC comprises blood plasma derived proteins. The term "derived" relates to be received from a source. For example, "is derived from" relates to taken from, obtained from, originates from, or received from.

In some embodiments of any aspect, the fibrinogen comprises plasma cryoprecipitated fibrinogen. In some embodiments, the fibrinogen originates (or is derived) from plasma cryoprecipitated fibrinogen. In some embodiments of any aspect, both Factor VIII, and fibrinogen are present or originate from cryoprecipitate, typically from a single cryoprecipitate.

The term "cryoprecipitate", or any grammatical inflection thereof, refers to a blood derived component which is obtained from frozen plasma prepared from whole blood, recovered plasma or from source plasma which is collected by plasmapheresis. A cryoprecipitate can be obtained when frozen plasma is thawed in the cold, typically at a temperature of 0-4°C, resulting in the formation of a precipitate that contains fibrinogen and factor XIII. The precipitate can be collected, for example, by centrifugation. Thus, in the context of the present disclosure, the term "cryoprecipitated fibrinogen" refers to fibrinogen obtained from frozen plasma, typically the latter prepared from whole blood (also denoted as fibrinogen is obtained by plasma cryoprecipitation).

In some embodiments of any aspect, cryoprecipitated fibrinogen is obtained when frozen plasma thawed in the cold, typically at a temperature of 0-4 °C, resulting in the formation of a precipitate that comprises predominantly the fibrinogen. In some embodiments, the cryoprecipitate is collected, for example by centrifugation and is then dissolved in a suitable buffer, such as, a buffer containing 120 mM sodium chloride, 10 mM trisodium citrate, 120 mM glycine, or 95 mM arginine hydrochloride.

In some embodiments of any aspect, the cryoprecipitated fibrinogen is derived from the biologically active component (BAC) of blood plasma. In some embodiments, the BAC is viral inactivated. There are several types of BAC.

In some embodiments of any aspect, BAC is Biologically active component 2 (BAC2), i.e. a biologically active component that lacks tranexamic acid. BAC2 is a concentrated viral-inactivated cryoprecipitate of human plasma (the cryoprecipitate is typically prepared as described in EP 534,178) which consists mainly of fibrinogen (approx. 85%) and is plasminogen-depleted (the removal of plasminogen is typically carried out as described in EP 1,390,485) and without anti-fibrinolytic agents added. In view of removal of plasmin/plasminogen from the cryoprecipitate, there is no need to add anti-fibrinolytic agents, such as tranexamic acid, aprotinin or the like.

Accordingly, in some embodiments of any aspect, the fibrinogen component is derived from BAC2 which is depleted from plasmin(ogen) and does not comprise tranexamic acid or aprotinin. In some embodiments of any aspect, the fibrinogen component is derived from BAC2 and does not contain anti-fibrinolytic agents. This is considered a second-generation BAC. During BAC2 preparation, plasminogen (the enzyme precursor of plasmin, which breaks down fibrinogen and fibrin) is removed. Further, in accordance with some embodiments, in addition to fibrinogen, the fibrinogen concentrate contains immunoglobulin (e.g., above 1% w/v) at least one, typically combination of two or more of fibronectin, albumin, and a residual protein (residual proteins in total up to 1% w/v from the total concentrate composition) selected from plasmin(ogen), vWF, Factor VIII, Antithrombin III, and serpine proteins. In some embodiments, the fibrinogen concentrate contains at least/not less than 40 g/L (4%) of clottable proteins i.e. proteins comprising mostly fibrinogen and including additional proteins such as Factor XIII - 2-9 IU/mL; Fibronectin - 0.5-6 mg/mL); and Albumin - 9-30 mg/mL.

In some embodiments of any aspect, the fibrinogen is a biologically active component of a plasma cryoprecipitate-derived from antihemophilic factor preparation. An Antihemophilic factor is a naturally occurring protein in the blood that helps blood to clot.

In some embodiments of any aspect, fibrinogen is purified from an aluminum hydroxide precipitate from a byproduct in the manufacture process of factor VIII (FVIII) as disclosed in WO2013/001524A1.

The BAC solution can further comprise stabilizers such as arginine, lysine and other sealant additives as known in the art. In some embodiments, BAC, and preferably BAC2, is derived from concentrated plasma cryoprecipitate.

Examples of fibrinogen sources include, but are not limited to, recombinant fibrinogen, plasma purified fibrinogen, including fibrinogen component of BAC2, fibrinogen component of Tisseel (containing aprotinin, an antifibrinolytic agent).

In some embodiments of any aspect, the blood derived fibrinogen concentrate is a byproduct of the manufacture process of factor VIII and may be selected from acid-precipitate, chill-precipitate, aluminum hydroxide precipitate (see, for example, US Patent no. 4,455,300), glycine precipitate (see, for example, US Patent no. 4,297,344), ethanol precipitate, and heparin precipitated paste.

In some embodiments of any aspect, plasma cryoprecipitated fibrinogen denotes, without being limited thereto, fresh frozen plasma precipitate following centrifugation containing total protein in the range of 30 to 60 mg/ml; total viable count (TVC) <1000 CFU/ml; Factor XIII, 2 to 9 IU/ml; Fibronectin - 0.5 to 6 mg/ml; and clottable Fibrinogen - 18 to 39 mg/ml. In exemplary embodiments, the composition comprises FXIII (e.g., about 0.6 IU/ml); fibronectin (e.g., 1.8 mg/ml); glycine (e.g., about 2.4 mg/ml); citrate (e.g., about 0.8 mg/ml); and arginine hydrochloride (e.g., about 10.4 mg/ml mg/ml).

In yet some other embodiments, the blood derived fibrinogen concentrate comprises or is suspended or precipitated Cohn Fraction I, at times, also referred to as "Paste I".

In some embodiments of any aspect, a BAC composition comprises one or more anti-fibrinolytic agents (e.g., tranexamic acid) and arginine hydrochloride.

In some embodiments of any aspect, the ratio of the positively charged amino acid arginine to Factor VIII, ranges from above 1.4 to below 8.3 mg/IU. In some embodiments, the ratio of the positively charged amino acid arginine to Factor VIII, ranges from above 1.5 to below about 8.2 mg/IU. In some embodiments, the ratio of the positively charged amino acid arginine to Factor VIII, ranges from 2 to 8 mg/IU. In some embodiments, the ratio of the positively charged amino acid arginine to Factor VIII, ranges from about 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, or about 8.2 mg/IU, including any value and range therebetween.

In some embodiments of any aspect, the arginine is present at a concentration ranging from above 7.5 mg/ml to below 30 mg/ml. In some embodiments, the arginine is present at a concentration ranging from about 8 mg/ml to about 28 mg/ml. In some embodiments, the arginine is present at a concentration of about 8, 10, 12, 14, 16, 18, 20, 22, 24, 26, 28, or about 30 mg/ml, including any value and range therebetween.

In some embodiments of any aspect, the arginine is present at a concentration ranging from above 7.5 mg/ml to below 30 mg/ml, without decrease of protein's biological functions.

In some embodiments of any aspect, the ratio of the positively charged amino acid e-g- arginine to Factor VIII, ranges from above 6 to below 47.5 mmol/IU (considering Molecular wt. of L-Arginine monohydrochloride is 210.66; and the FVIII drug substance ranges from about 3-6 IU/ml). In some embodiments, the ratio of the positively charged amino acid arginine to Factor VIII, ranges from above 10 to below about 45 mmol/IU. In some embodiments, the ratio of the positively charged amino acid arginine to Factor VIII, ranges from 10 to 45 mmol/IU. In some embodiments, the ratio of the positively charged amino acid arginine to Factor VIII, is about 7, 8, 9, 10, 11, 12, 13, 14, 15, 16. 17, 18, 19, 20, 21, 22, 23, 24, 25, 26, 27, 28, 29, 30, 31, 32, 33, 34, 35, 36, 37, 38, 39, 40, 41, 42, 43, 44, 45, or about 47 mmol/IU, including any value and range therebetween.

In some embodiments of any aspect, the arginine is present at a concentration ranging from above 7.5 mg/ml to below 30 mg/ml. In some embodiments, the arginine is present at a concentration ranging from about 8 mg/ml to about 28 mg/ml. In some embodiments, the arginine is present at a concentration of about 8, 10, 12, 14, 16, 18, 20, 22, 24, 26, 28, or about 30 mg/ml, including any value and range therebetween.

In some embodiments of any aspect, the arginine is present at a concentration ranging from above 7.5 mg/ml to below 30 mg/ml, without decrease of protein's biological function(s).

Herein "proteins" may refer to e.g., fibrinogen, Factor VIII, or both. The term "protein's biological functions" as used herein refers to an activity that is natural to the relevant protein, as tested in vitro or in vivo. By "without decrease" it is meant to refer to less than about 30%, less than about 20%, or less than about 10% decrease.

In some embodiments of any aspect, the arginine is present at a concentration ranging from above 7.5 mg/ml to about 10 mg/ml. Reference is made to the Examples section (and to Figures 1A-1B) showing that in the range of above 7.5 to less than 10 mg/ml the effect of the arginine on proteins (i.e. fibrinogen and Factor VIII in viral inactivate lyophilized plasma in the form of cryo concentrate) was not significant and the reconstitution time was significantly shorter than with lower concentration of arginine. Sodium chloride may be added to meet the physiological osmolarity range of about 200 to about 400 mOsm/kg, or, in some embodiments, about 250 to about 350 mOsm/kg.

The term "osmolarity" refers to the concentration of osmotically active solutes in solution. As used herein, the term "a physiological osmolarity" refers to an osmolarity in accord with, or characteristic of, the normal functioning of a living organism.

In exemplary embodiments, the weight ratio of sodium chloride to fibrinogen ranges from 1:3 to 1:5, respectively.

In some embodiments of any aspect, the arginine is present at a concentration ranging from about 8 mg/ml to about 28 mg/ml. In some embodiments, the arginine is present at a concentration of about 8, 10, 12, 14, 16, 18, 20, 22, 24, 26, 28, or about 30 mg/ml, including any value and range therebetween.

In some embodiments of any aspect, the ratio of arginine to fibrinogen ranges from 0.1 to below than about 1.6, respectively. In some embodiments, the ratio of arginine to fibrinogen ranges from 0.3 to about 1.6, respectively.

According to the claims, the ratio of arginine to total protein ranges from above 0.2 mg per mg protein to below 1 mg of arginine per mg protein. In some embodiments of any aspect, the ratio of arginine to total protein ranges from above 0.2 mg per mg protein to about 0.4 mg of arginine per mg protein. In some embodiments of any aspect, the ratio of arginine to total protein is 0.25, 0.3, 0.35, 0.4, 0.45, 0.5, 0.55, 0.6, 0.65, 0.7, 0.75, 0.8, 0.85, 0.9, 0.95, or below 1 mg of arginine per mg protein, including any value and range therebetween. Reference is made to the Examples (and to Figure 1B) showing that a narrow ratio of arginine to protein enables fast reconstitution without loss of activity.

In some embodiments of any aspect, the composition is a pharmaceutical composition. As used herein, the term "pharmaceutical composition" refers to a substance or mixture of substances for administration to a subject. In the context of the present disclosure, the freeze dried and virus-inactivated cryoprecipitable proteins which, once reconstituted in a liquid pharmaceutically compatible medium, such as pure water for injections, can be directly injected to a patient. In some embodiments of any aspect, the pharmaceutical composition comprises one or more excipients. As used herein the term "excipient" refers to a substantially inert substance which is included in a pharmaceutical composition. Excipients can be added, for example, in order to ensure that the active substances of the composition retain their chemical stability and/or biological activity upon storage, to aid the manufacturing process and/or for aesthetic reasons e.g., color.

The pharmaceutical composition may comprise one or more pharmaceutically acceptable carriers. The term "pharmaceutically acceptable carrier" refers to any diluent or a vehicle which is suitable for human or other animal use. E.g., "a pharmaceutically acceptable carrier or diluent" refers to reagents, compounds, materials, compositions, diluents that are compatible with the constituents in the formulation and suitable for use in contact with the tissues of human beings and animals without excessive toxicity, irritation, allergic response, or other complication commensurate with a reasonable benefit/risk ratio. A pharmaceutically acceptable carrier suitable for use with the formulation disclosed herein includes liquids, semisolid and solid materials.

In some embodiments, the pharmaceutical composition is in the form of a powder. In some embodiments, the pharmaceutical composition is in the form of a powder suspended in an aqueous medium. In some embodiments, the pharmaceutical composition is a ready-for-use composition. The term "ready-for-use", as used herein, refers to a form of a composition which allows its prompt and direct use without further process, such as reacting or mixing ingredients other than dissolving ingredients in a carrier.

Human blood-derived products may carry a risk of transmitting infectious agents such as viruses. Effective reduction of viral transmission risk can be achieved by including at least two orthogonal viral inactivation steps. Yet, including additional steps in the manufacture of e.g., a platelet extract may compromise the recovery and activity of the factors contained therein. One of these methods of viral inactivation is known as "Solvent Detergent (S/D) viral inactivation treatment".

Accordingly, in some embodiment of any aspect of the present disclosure, the composition is viral activated. The composition may be virus inactivated, preferably using the solvent detergent inactivation technique, and optionally is doubly inactivated by the combination of the solvent detergent method with a second virus inactivation method, for example, with pasteurization or inactivation with UVC light of the fibrinogen solution, typically, prior to a lyophilization step.

Although the heat treatment of virus inactivation allows to obtain a virus-free freeze-dried product, the process may comprise, typically, prior to a step of addition of the stabilizing and solubilizing formulation to a liquid composition of cryoprecipitable proteins, at least one additional virus inactivation and/or elimination step of the liquid compositions by solvent-detergent and/or nanofiltration, for instance on filters of 35 nm, in order to finally ensure a total and complete inactivation and elimination of viruses.

In some embodiments, the implementation of the viral inactivation process leads to freeze dried and virus-inactivated cryoprecipitable proteins which, once reconstituted in a liquid pharmaceutically compatible medium, such as pure water for injections, can be directly injected to a patient. This therapeutic quality of cryoprecipitable proteins is obtained thanks to the stabilizing and solubilizing formulation, which enables the use of all the above-mentioned treatments, especially the virus inactivation and elimination treatments, whereas the biological activity of these treated proteins and the dissolution characteristics of the dried form are maintained. The stabilizing and solubilizing formulation, used according to the process of the invention, applies to cryoprecipitable proteins, such as Factor VIII, von Willebrand Factor, Factor XIII, fibrinogen and fibronectin, obtained by methods of fractionation of blood plasma known to those skilled in the art.

According to specific embodiments, the composition comprising same described herein are formulated for parenteral (e.g., intravenous) administration, e.g., by bolus injection or continuous infusion.

According to specific embodiments, the pharmaceutical composition is administered by intravenous (IV) infusion. According to specific embodiments, the composition is administered within minutes using an electronic infusion pump; e.g. over a time period of 1 - 5 minutes.

In some embodiments, the pharmaceutical composition is for IV administration (e.g., by injection) to a subject in need thereof, in a dose of up to about 60 mg per kg of body weight, as needed.

Alternatively, the dose is individually calculated for each patient based on the target plasma fibrinogen level based on the type of bleeding, actual measured plasma fibrinogen level and body weight using the following formula:

Dose (mg/kg body weight) = [Target level (mg/dL) - measured level (mg/dL)]/1.8 (mg/dL per mg/kg body weight).

Typically, but not exclusively, the dose is dissolved in an aqueous medium at about 1 mg solid per 30 to 60 ml, e.g., 40-50 ml.

According to specific embodiments, the IV infusion is a fast-dripping IV infusion, e.g., over a time period of less than 30 minutes, e.g., over a time period of less than 10 minutes, e.g., over a time period of about 5 minutes.

According to other specific embodiments, the IV infusion is a slow dripping IV infusion e.g., over a time period of more than 30 minutes.

According to specific embodiments, the compositions may be suspensions, solutions or emulsions in oily or aqueous vehicles.

Compositions for parenteral administration include aqueous solutions of the active preparation in water-soluble form. Additionally, suspensions of the peptides may be prepared as appropriate oily or water-based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the peptides to allow the preparation of highly concentrated solutions.

Alternatively, the active composition may be in a powder form for constitution with a suitable vehicle, e.g., sterile, water-based solution, before use.

The compositions may also contain formulatory agents such as suspending, stabilizing and/or dispersing agents.

As used herein the term "subject" refers to a human subject at any age and of any gender.

In an aspect of the present disclosure, there is provided a unit dosage form comprising fibrinogen, Factor VIII, and a polar or basic amino acid, wherein the ratio of the polar or basic amino acid to Factor VIII ranges from above 1.4 to below 8.3 mg/IU, or 6 to 47.5 mmol/IU formulated for intravenous (IV) administration. In some embodiments, the unit dosage form is substantially devoid of hydrophobic amino acid. Embodiments of polar and basic amino acids are described hereinthroughout and are incorporated herein. The claims demand a positively charged amino acid which comprises arginine, wherein the ratio of arginine to total protein ranges from above 0.2 mg per mg protein to below 1 mg of arginine per mg protein.

In some embodiment of any aspect of the present disclosure, the composition further comprises von Willebrand Factor. In some embodiment of any aspect of the present disclosure, the composition further comprises Factor XIII.

In some embodiment of any aspect of the present disclosure, the composition comprises about 5% to about 25% albumin, by weight of the total proteins. In some embodiment of any aspect of the present disclosure, the composition comprises about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, or about 25% albumin, by weight of the total proteins, including any value and range therebetween.

In some embodiments of any aspect of the present disclosure, the composition is substantially devoid of added albumin.

By "added" it means, other than naturally presented e.g., in the cryoprecipitate.

In some embodiments of any aspect of the present disclosure, the composition is in the solid form. Non-limiting examples of forms of solid compositions (which may be dissolved using the methods and devices described herein) include, but are not limited to, lyophilized "cakes", solid particles, particle dispersion, powder, and flakes. Solid compositions may be produced having different protein densities.

Thus, in some embodiments, the disclosed composition, e.g., upon applying lyophilization process is in the form of a cake.

The three-dimensional fibrinogen "cake" may me expressed as mg/cm$^3$, is substantially equivalent to the protein concentration in the solution in mg/ml prior to lyophilization. Hence, dilution of a disclosed composition in the form of stock solution prior to lyophilization results in a volume of the "cake" which is substantially equal to the volume of the solution from which the "cake" may be prepared. Therefore, typically dissolution of the "cake" in a volume that is equal ($\pm$ about 20%) to the volume of the "cake" results in a solution having a concentration equivalent ($\pm$ about 20%) to that of the solution from which were dissolved in aqueous solvent and the level of protein dissolution is measured as described below.

Highly porous cakes with large surface area may be used for shortening the dissolution time, since collapse of such cakes made by e.g., a plunger in syringe barrel, prior to the introduction of the solvent into syringe barrel is prevented. Each of the low-density "cakes" obtained from the diluted solutions may be dissolved in 30 to 50, e.g., about 40 ml aqueous solution at a room temperature (20 to 30 °C).

In some embodiments of any aspect of the present disclosure, the composition is in a lyophilized or freeze-dried form. In some embodiments of any aspect of the present disclosure, the composition is in the form of a cake.

The term "freeze dried" as it is employed herein is preferably, but not by way of limitation, intended to refer to the practice of lyophilizing the free water present in a solution. However, it should be understood that the term "freeze drying" in the sense of the present disclosure is intended to embrace a practice which may involve removal of a substantial though not a majority of the water present e.g., by sublimation.

As used herein, the term "lyophilized" or any inflection thereof, refers to a product obtained by a process of freezing a solution and then reducing the concentration of water e.g., by sublimation to levels which do not support biological or chemical reactions.

Such as a lyophilized "cake" having low protein density, in an aqueous solvent, results in higher dissolution level. In order to dissolve the "cake" in the aqueous solvent, a solid dispersion can optionally be prepared by mechanically crushing the "cake". In order to dissolve the "cake" in the aqueous solvent, a solid dispersion can optionally be prepared by mechanically crushing the "cake". The dispersion can optionally be introduced into a milling device to produce fine powder as described e.g., in WO2008/053475.

In some embodiments, components i.e., fibrinogen, Factor VIII, and a positively charged amino acid comprising arginine are present in a single powder.

As used herein, the term "cake" or "solid cake" refers to a porous and spongy structure-like composition resulting from the lyophilization process, while maintaining substantially the same volume of the corresponding liquid solution. It is noted that a "solid cake" can substantially support its own structure without collapsing. As used herein, the term "collapse" with regard to a cake refers to the point at which the cake can no longer support its own structure. In some embodiments, the cake is in the amorphic structure. In some embodiments, the cake is in the sponge-like structure. The inventors have surprisingly found that the structure (e.g., sponge-like structure) is in a monolithic structure, typically in a uniform (also referred to as "homogeneous") structure can be obtained upon using a certain amount of the positively charged amino acid arginine. Reference is made in this regard to Table 5 in the Examples section showing that at a ratio of the positively charged amino acid arginine to Factor VIII ranging from above 6 to below 47.5 mmol/IU, a homogenous and crystalline cake is obtained. As used herein, the term "monolithic" refers to a composition, or its structure, that is not composed of two or more distinct macroscopic layers.

As used herein, the term "solid" or "solid composition" refers to a composition having a water content of equal to or less than about 5% (w/w) water such as equal to or less than 3%, based on the total weight of the solid composition. The term "crystalline", as used herein refers to a solid phase in which the material has a regular and uniformly ordered internal structure.

The term "liquid" relates to a substance that can flow, has not fixed shape, and is not a solid or gas. The term "solution" relates to dispersed or dissolved substance(s) in the medium in which it is dispersed or dissolved or to a single homogeneous liquid phase that is a mixture in which the components are uniformly distributed throughout the mixture.

In some embodiments of any aspect of the present disclosure, the composition is in the solid form e.g., cake or crushed cake, and is characterized by being dissolved within less than 30 min. In some embodiments of any aspect of the present disclosure, the composition is in the solid form, and is characterized by being dissolved within less than 20 min. In some embodiments of any aspect of the present disclosure, the composition is in the solid form and is characterized by being dissolved within less than 10 min. In some embodiments of any aspect of the present disclosure, the composition is in the solid form, and is characterized by being dissolved within less than 5 min. In some embodiments of any aspect of the present disclosure, the composition is in the solid form, and is characterized by being dissolved within 1 to below 5 min. In some embodiments of any aspect of the present disclosure, the composition is in the solid form, and is characterized by being dissolved within 1 to about 4 min. In some embodiments of any aspect of the present disclosure, the composition is in the solid form, and is characterized by being dissolved within 0.5 to about 3 min.

Hereinthroughout, by "dissolved", or any inflection thereof, at an atmospheric pressure and 25 °C in an aqueous solvent, typically water, or, in some embodiments, saline, at an atmospheric pressure without an aggressive mixing or blending. The term "dissolved" in the meaning of the present disclosure refers to systems in which no discrete solid particles, foaming and/or turbidity are observed by a naked eye in the solvent. As used herein, the terms "dissolving", "solubilizing", and "reconstituting" and their respective grammatical inflections may be interchangeable.

As used herein, the term "atmospheric pressure" refers to the force per unit area exerted on a surface by the weight of air above that surface at a given location. Standard sea-level atmospheric pressure is 1 atmosphere, or 1000 mBar.

The dissolution level in the solution formed by dissolving the solid fibrinogen composition in aqueous solvent can be measured as described below, or by methods known in the art.

In some embodiments of any aspect of the present disclosure, the composition is substantially devoid of hydrophobic amino acid or, in some embodiments, of added hydrophobic amino acid.

As used herein the term "hydrophobic amino acid" refers to an amino acid exhibiting hydrophobic properties such as hydrophobicity of greater than zero according to the normalized consensus hydrophobicity scale of Eisenberg, 1984, J. Mol. Biol. In some embodiments, hydrophobic amino acid is selected from alanine, cysteine, phenylalanine, glycine, histidine, isoleucine, leucine, methionine, threonine, valine, tryptophan, tyrosine, proline, or any combination thereof. In some embodiments, hydrophobic amino acid is selected from alanine, cysteine, phenylalanine, histidine, isoleucine, leucine, methionine, threonine, valine, tryptophan, tyrosine, proline, or any combination thereof.

According to an aspect of the present invention, there is provided a method for obtaining a reconstituted solid fibrinogen in an aqueous medium, the method comprising providing the solid lyophilized composition as disclosed herein; and adding an aqueous medium at a volume ranging from above 100% to less than 170%, e.g., about 125%, of the solid composition. Reference is made to the Examples section below (see Table 6) showing, surprisingly, that reconstitution time in 50 ml of the larger volume of 40 cm$^3$ is shorter than that of the 30 cm$^3$ and the fibrinogen activity measured by Clauss is higher.

It is noteworthy that the volume of the lyophilization samples ("filling volume") is preferably less than 50 ml to avoid extending the lyophilization time.

The composition disclosed herein may be used for any therapeutic purpose. The term "any therapeutic purpose" refers to any curative or preventive treatment in a subject. Exemplary therapeutic purposes include, but are not limited to, sealing a bore hole formed in a tissue or organ e.g., a bone; anastomosis at blood vessels; joining tissue parts e.g., soft tissue parts; treating or preventing dura defects e.g., tears and leaks following dural injections, fissures or cracks; treating or preventing bleeding; treating or preventing air leaks such as following pulmonary lung resection; treating or preventing defects following intestinal perforation; treating or preventing defects following anastomosis procedure carried out in any tissue e.g., uterine, esophagus, stomach, pancreas, pancreatic duct, gall bladder, bile duct, intestinal (including the small intestine and the large intestine), and rectum; treating or preventing post-operation leaks in any tissue e.g., uterine, esophagus, stomach, pancreas, pancreatic duct, gall bladder, bile duct, intestinal (including the small intestine and the large intestine), and rectum; preventing or diminishing the occurrence of post-operative leaks at the staple or suture line e.g., by applying the powdered composition according to the invention, either alone or combined with a matrix e.g., a patch, onto at least a part of a defect such as a staple/suture line; for strongly affixing prosthesis e.g., during a hernia operation; for staple/suture line reinforcement; to prevent or diminish alveolar air leakage; treating or preventing renal defects; treating or preventing fistulas; treating or preventing heart defects e.g., penetrating heart wounds; reinforcing of a vascular graft prosthesis; and treating or preventing cerebrospinal fluid leakage. In some embodiments, the composition, or matrix according to any of the embodiments discloses herein is for use in providing hemostasis, sealing leaks and/or joining structures. In some embodiments, there is provided a method of providing hemostasis, sealing leaks and/or joining structures in a subject in need thereof, the method comprising use of the composition or the matrix according to any of the embodiments disclosed herein.

As used herein, the term "bleeding" refers to extravasation of blood from any component of the circulatory system. A "bleeding" thus encompasses unwanted, uncontrolled and often excessive bleeding in connection with surgery, trauma, or other forms of tissue damage, as well as unwanted bleedings in patients having bleeding disorders, and severe bleeding after birth, including hemorrhage associated with caesarian section. The term "blood", or any grammatical inflection thereof, also includes blood fractions, such as plasma. "Wound" as used herein refers to any damage to any tissue of a patient which results in the loss of blood from the circulatory system and/or any other fluid from the patient's body. The damage may have been caused by any agent or source, including traumatic injury, infection or surgical intervention. A wound may be in a soft tissue, such as an organ, or in hard tissue, such as bone. The tissue may be an internal tissue, such as an organ or blood vessel, or an external tissue, such as the skin. The loss of blood may be internal, such as from a ruptured organ, or external, such as from a laceration.

As used herein, and unless stated otherwise, the terms "weight ratio", "by weight", "w/w", "weight percent", or "wt. %", which are used herein interchangeably describe the concentration of a particular substance out of the total weight of the corresponding mixture, solution, suspension, formulation or composition. As used herein, and unless stated otherwise, the terms "volume ratio", "by volume", "v/v", "volume percent", or "v %", which are used herein interchangeably describe the concentration of a particular substance out of the total volume of the corresponding mixture, dispersant, solution, suspension, formulation or composition.

In some embodiments, the composition is stable.

The terms "stable", and "stability" when referring to the disclosed composition (either as a solid or being dissolved in a solution), mean that an active component within, e.g., fibrinogen at a certain temperature and after certain time duration remains at least 70 % active, that is, capable of forming a fibrin clot.

The powder may be an intermediate suitable for combination with another powder or material, suitably to produce a sterile final composition or material. Compositions described herein, as well as the contents of the abovementioned kits, may, if desired, be presented in a pack or dispenser device, which may contain one or more unit dosage forms containing the composition (e.g., fibrinogen) and additional ingredients and/or reagents (e.g., dispersant) for preparing the composition. The pack may, for example, comprise metal or plastic foil, such as a blister pack. The pack or dispenser device may be accompanied by instructions for administration. The pack or dispenser device may also be accompanied by a notice in a form prescribed by a governmental agency regulating the manufacture, use, or sale of pharmaceuticals, which notice is reflective of approval by the agency of the form of the compositions for human or veterinary administration. Such notice, for example, may include labeling approved by the U.S. Food and Drug Administration (FDA) for prescription drugs or of an approved product insert. Compositions comprising a preparation of the invention formulated in a pharmaceutically acceptable carrier may also be prepared, placed in an appropriate container, and labeled for use for an indicated application and/or for treatment of an indicated condition, as further detailed herein. The term "preparation" refers to a physiologically suitable for therapeutic use.

It will be appreciated that compositions of embodiments of the present disclosure may be attached to or included in medical devices, such as for promoting wound healing.

In another aspect, there is provided a method for obtaining a highly soluble solid composition comprising fibrinogen and Factor VIII, the method comprising adding a polar or basic amino acid to a liquid composition comprising the fibrinogen and Factor VIII in a ratio of the polar or basic amino acid to Factor VIII ranging from above 1.4 to below 8.3 mg/IU, respectively; and drying the liquid composition so as to obtain the solid composition, wherein the claims demand a positively charged amino acid comprising arginine and a ratio of arginine to total protein ranging from above 0.2 mg per mg protein to below 1 mg of arginine per mg protein.

Embodiments of fibrinogen, polar or basic amino acid, solubility are described hereinthroughout and are incorporated herein.

In some embodiments, there is provided a solid composition obtained by the method for obtaining a highly soluble solid composition. Embodiments of the composition described hereinthroughout and are incorporated herein to a solid composition obtained by the method for obtaining a highly soluble solid composition.

In another aspect of the present disclosure, the disclosed composition in any aspect or embodiment thereof is for use in a method for preparing a fibrin sealant in/on an injured tissue of a subject, e.g., by applying the disclosed composition in any aspect and/or embodiment thereof on a surface of the tissue.

In another aspect of the present disclosure, there is provided a method for preparing a fibrin sealant in/on an injured tissue of a subject, e.g., by applying the disclosed composition in any aspect and/or embodiment thereof for a hemostatic treatment as a hemostat on a surface of the tissue.

The term "hemostatic" refers to an ability to prevent, reduce, or stop blood loss e.g., from wounds, such as surgical or traumatic wounds, e.g., by promoting blood clot formation. Hemostasis" (or "haemostasis") refers to the first stage of wound healing. It is a process which causes bleeding to stop. By "assist in hemostasis" it means to help reduce or stop bleeding. By "applied to a bleeding tissue" it is meant to refer to e.g., a topical application of the composition at the bleeding site, e.g., at a surgical site to control bleeding. Control of bleeding is needed in various situations including treatment of wounds.

As used herein, the terms "controlling", "preventing", or "reducing", which may be used herein interchangeably in the context of the bleeding, including any grammatical inflection thereof, indicate that the rate of the blood extravagated is essentially nullified or is reduced e.g., by 10 %, at least 20 %, at least 30 %, at least 40 %, at least 50 %, at least 60 %, at least 70 %, at least 80 %, at least 90 %, or even by 100 %, of the initial rate of bleeding, compared to situation lacking the contact of the disclosed composition in/on the bleeding site. Methods for determining a level of appearance of bleeding are known in the art.

Any aspect and embodiment of the hereinthroughout disclosed composition may be incorporated to the aspect and embodiments of the kit, including embodiments of the composition, the dispersant, and/or the powder.

Optionally, the disclosed fibrinogen solid as well as a thrombin powder are each packaged individually (e.g., in a dry form or in a solution) in the kit. Thus, each of the two ingredients is packaged in separate packaging material, in addition to the packaging material of the whole kit.

Alternatively, the fibrinogen and the thrombin are packaged together (e.g., as a single dry powder) in the kit in the same packaging material, and optionally, the dispersant is provided in an additional container. In some embodiments, the kit further comprises instructions on how to combine the ingredients of the kit and/or how to combine the ingredients of the kit with an additional ingredient (e.g., a dispersant), in order to produce the desired composition.

Optionally, the kit further comprises a dispersant. Dispersant such as e.g., glycerol may be in a pure form or in a solution with another liquid (e.g., water, saline or aqueous buffer). Optionally, the dispersant is packaged individually, apart from the powder(s). Alternatively, the dispersant is packaged in combination with the powder(s), for example, as a ready-for-use composition described herein. In such embodiments, the kit may further contain a measuring means, e.g., a measuring cylinder, to measure the volume of the dispersant or a component thereof.

Additionally or alternatively, the hemostatic kit may comprise an applicator such as syringe- or injector-containing the blend, mixture or powder and another syringe containing the dispersant. For example, dual-syringe mixing devices may produce a substantially homogenous paste mixture, solition or a formulation by combining initially separate liquid and powders and then passing the blended contents back and forth between two connected syringes via interconnected outlet(s). Therefore, a low expression force for dispensing the paste from a syringe may be preferred for ease of mixing and ultimately for deployment of the resulting paste, solution or formulation. The desired expression force may be less than 1.51 lbf.

In some embodiments, at least one of the containers in the kit is a pre-filled syringe. In some embodiments, a syringe is provided in addition to the container(s) of the kit. In some embodiments, the container is in a specific type, such as a vial or an applicator such as a syringe.

In some embodiments, at least one of the containers in the kit is a pre-filled syringe. In some embodiments, a syringe is provided in addition to the container(s) of the kit. The term "container" may refer to any generic structure such as a vessel or a vial that may contain the paste.

The kit may be applied using an applicator device which may be used for administering several and sequential injections of the composition. In one embodiment, the applicator device enables multiple injections of a fixed-dose of the mixed components on a 2-D surface of a tissue while moving the device. In one embodiment, the applicator has a syringe with an injection needle, which is optionally automatically retracted from the patient's skin after the injection is completed without the need for the administrator to lift the device upward from the injection surface. In one embodiment, the kit may be used for the administration of a sealant.

The hemostatic kit of the invention may be a kit for use in reducing, preventing or stopping blood flow, e.g., in open wounds, and it may be used for reducing, preventing or stopping blood flow during a procedure, such as during, before, or after a surgical procedure such as, for example, laparoscopic surgery, neurosurgery, abdominal surgery, cardiovascular surgery, thoracic surgery, head and neck surgery, pelvic surgery and skin and subcutaneous tissue procedures. The kit may be used for reducing or preventing blood flow from the skin, or in internal organs.

In one embodiment, this kit may be stored at around the room temperature, such as at a temperature in the range of 8 to 40 °C, or at lower temperatures.

In some embodiments of any aspect of the kit or the composition disclosed herein, the powder or blend may comprise an additive e.g., calcium salt and/or one or more excipients, e.g., selected from, without being limited thereto, one or more amino acids, saccharides, and/or saccharide derivatives.

The term "additive" is meant to be understood as any substance that can be added to a composition, and may also include an active additive such as calcium salt as described below.

The term "excipient" as used herein denotes a non-active or non-therapeutic agent added to a pharmaceutical composition e.g., to provide a desired consistency or stabilizing effect. Excipients can be added, for example, in order to ensure that the active substances of the composition retain their chemical stability and/or biological activity upon storage, to aid the manufacturing process and/or for aesthetic reasons e.g., color.

Calcium is an important element in the clotting cascade, and may be needed for activation of factor XIII into factor XIIIa, which cross-links and stabilizes fibrin to generate an insoluble clot.

Accordingly, in some embodiments of any aspect of the disclosed kit and/or composition, the blend, mixture or powder further comprises an additive such as, without limitation, calcium. Calcium used with the invention may be in the form of a salt, e.g., calcium chloride salt. Alternatively, additional salts may be used, such as calcium acetate and/or calcium citrate. In the kit, the calcium salt may be provided in the composition comprising the powder. Alternatively, the excipient(s), and/or the calcium salt, may be provided in the kit in a separate container, or the excipient(s), and/or the calcium salt, may be provided in the kit in the same container comprising the blend/mixture/powder component.

In some embodiments of any aspect of the kit and/or compositions, the disclosed composition may be used in conjunction with a backing, pad, bandage, gauze, sponge, scaffold, or matrix to provide mechanical strength to cover the wound surface. In this case, the instant matrix may be also supported on a pad for ease of application or tamponade.

In some embodiments of any aspect of the kit and/or compositions, the composition is sterile. Especially when handling blood products, the sterility issue is crucial, and specifically the issue of viral inactivation. As described herein, in general, viral inactivation may be carried out by any number of methods, including solvent detergent, heat inactivation, irradiation, and nanofiltration. Typically, the standard for viral inactivation requires using two different (orthogonal) methods. Additionally, FDA standard for sterility requires filtration.

The term "sterile" as used herein means having a low bioburden, effectively being germ-free, e.g., essentially or even absolutely being free from microorganisms, e.g., bacteria and viruses. Sterilization is the process of reducing the bioburden to an effectively germ-free level.

As used herein the term "about" refers to $\pm$ 10 %.

The terms "comprises", "comprising", "includes", "including", "contains", "containing", "has", "having", and their conjugates mean "including but not limited to". The term "consisting of" means "including and limited to". The term "consisting essentially of" means that the composition, method or structure may include additional ingredients, steps and/or parts, but only if the additional ingredients, steps and/or parts do not materially alter the basic and novel characteristics of the claimed composition, method or structure.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment of the invention may include a plurality of "optional" features unless such features conflict.

As used herein, the singular form "a", "an", and "the" include plural references unless the context clearly dictates otherwise. Thus, as used herein, the indefinite articles "a" and "an" mean "at least one" or "one or more" unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the terms "method" or "process" which may be used herein interchangeably refer to manners, means, techniques and procedures for accomplishing a given task including, but not limited to, those manners, means, techniques and procedures either known to, or readily developed from known manners, means, techniques and procedures by practitioners of the chemical, analytical, pharmacological, biological, biochemical and medical arts.

In those instances where a convention analogous to "at least one of A, B, and C, etc." is used, in general such a construction is intended in the sense one having skill in the art would understand the convention (e.g., "a composition having at least one of A, B, and C" would include but not be limited to compositions that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A", "B", or "A and B".

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following non-limiting examples.

## EXAMPLES

Reference is now made to the following examples, which together with the above descriptions illustrate some embodiments of the invention in a non-limiting fashion.

In the following examples Plasma Cryoprecipitate Concentrate (denoted as "cryo" or "cryoprecipitate") are plasminogen depleted and viral inactivated. Specifically, the cryoprecipitate is a fibrinogen solution which is a Biological Active Coagulant (or Component) 2 (BAC2) drug substance (DS) was used.

**Example 1: Reconstitution Time of Lyophilized Cryo Originated from BAC2**

This step generates samples based on BAC2 with 20 mg/ml of arginine (Arg). These samples were further formulated by arginine and/or sodium chloride to obtain solutions of various formulations. In all the BAC2 formulation the pH is finally adjusted to 6.8-7.1. The solution is pre-filtered to 1 $\mu$m before formulation to avoid any aggregate or clots formation. The filtered formulation is finally filtered by a microbial filter before adjusted to the desired formulation and put into the lyophilizer.

In exemplary procedures, BAC2 samples DS, supplemented with 7.5 mg/ml final concentration of arginine was diluted 1:1.67 with aqueous solutions containing 11.29 g/L NaCl. The preparation of each cryo bottle was targeted to meet a final fibrinogen amount of 1 g per bottle/sample based on the following scheme: 15 ml of BAC2 sample (~ 70 mg/ ml Clottable Fibrinogen x 15 ml = 1050 mg Fibrinogen).

Practically, lyophilized cryo concentrate BAC2 was formulated by 7.5 mg per ml (300 mg per 1 gr of fibrinogen) with a total volume of 40 ml. This was carried out via a simple dilution of the BAC2 with and addition of 25 ml PuW (pure water) and adjusting the sodium chloride concentration to 11-12 g/L.

The experiment was carried out on three samples. The formulated bottles were lyophilized in Christ Martin Epsilon 2-12D LSCplus (Christ, Osterode am Harz, Germany) in 100 ml bottles (SGD, Type 1, non-siliconized, SGD Pharma Puteaux, France).

Lyophilization cycle was carried for a total of 83 hours with 8 process phases (see Table 1).

**Table 1:** Lyophilization cycle of Chris Martin lyo with Cryo concentrate in 100 ml bottles, Filled up to 42 ml Formulations

| Step | Process phase | Time (h:m) | Temperature °C | Vacuum (mbar) |
|------|--------------|-----------|---------------|---------------|
| 1 | Start value | 0:00 | 20 | OFF |

(continued)

| Step | Process phase | Time (h:m) | Temperature °C | Vacuum (mbar) |
|------|---------------|------------|----------------|---------------|
| 2 | Freezing | 1:00 | -30 | OFF |
| 3 | Freezing | 6:00 | -30 | OFF |
| 4 | Preparation | 0:30 | -25 | 0.120 |
| 5 | Sublimation | 1:00 | 10 | 0.120 |
| 6 | Sublimation | 56:30 | 10 | 0.120 |
| 7 | Sublimation | 0:30 | 25 | 0.012 |
| 8 | Secondary drying | 17:30 | 25 | 0.012 |
| | Total | 83:00 | | |

At the end of the lyophilization cycle the battles were closed and equilibrated with nitrogen to atmospheric pressure. The reconstitution time of each product was then tested.

As can be depicted in Table 2, the reconstitution time of the product containing about 300 mg of arginine per gram fibrinogen (7.5 mg/ml at a 40-42 ml) was ≥ 8 min.

**Table 2:** Reconstitution Time Results of Lyophilized Fibrinogen Samples comprised of 300 mg Arginine (or 7.5 mg per ml)

| BAC Drug substance Batch #* | Sample | Dissolution Time (min) | Mean Dissolution Time (min) |
|-----------------------------|--------|------------------------|------------------------------|
| 1 | #1 | 9:55 | 9:49 |
| 1 | #2 | 9:30 | 9:49 |
| 1 | #3 | 10:02 | 9:49 |
| 2 | #1 | 9:59 | 9:26 |
| 2 | #2 | 9:31 | 9:26 |
| 2 | #3 | 8:48 | 9:26 |
| 3 | #1 | 8:03 | 8:10 |
| 3 | #2 | 7:56 | 8:10 |
| 3 | #3 | 8:31 | 8:10 |
| | Average ± SD | | 9:08±0:49 |

*Three batches were used, originated from BAC2 DS at the different days. Each batch represents an independent run.

## Example 2: Effect of Arginine Concentration on Resuspension Time of Lyophilized Cryoprecipitate, and on Factor VIII Activity

The experiment was aimed to assess the effect of arginine concentrations at a fixed sodium chloride concentration on the reconstitution time and recovery of coagulation proteins of lyophilized, viral inactivated and plasminogen depleted cryoprecipitate (BAC2). For this purpose, the arginine was first depleted from BAC2 samples via intensive uses of ultrafiltration against the buffer without arginine (the original amount of arginine in BAC2 samples is 20 mg/ml).

The arginine depleted BAC2 sample was divided to 5 samples and each of them was adjusted with similar final concentration of NaCl and a gradual increasing amount of arginine: 0, 10, 30 50, 70 mg/ml. All 5 types of samples underwent the lyophilization. The obtained lyophilized cryoprecipitates were characterized for the cake appearance and processed to the dissolving in 50 ml water while the reconstitution time (including the rate of foaming) for each sample type was measured. The dissolved samples also were assessed for pH, osmolarity. Fibrinogen activity was assessed by Clauss method, and FVIII was assessed using clotting assay.

The materials and sample preparations are detailed below (solution preparations):

**1)** NaCl 1M solution- 116.8g NaCl were added to 2L PuW, stirred until all material was dissolved.

**2)** NaOH 0.5M solution- 60g were added NaOH to 3L PuW, stirred until all material was dissolved.
**3)** NaCl Diluent (final conc. of 5.64 g/L in reconstituted product) - 6.42g NaCl were added to 1L PuW, stirred until all material was dissolved.
**4)** Replacing buffer.

Table 3 summarizes the sample preparations.

**Table 3:** Sample Preparation; Starting Material: BAC2 batch

|  | Step | Required per 5L |
|---|---|---|
| 1 | Fill PuW into a sterile bottle, containing magnetic stirrer | 3.5L |
| 2 | Add Tri-Sodium Citrate dihydrate | 14.7g |
| 3 | Add Glycine | 45 g |
| 4 | Add Calcium Chloride dihydrate | 0.75 g |
| 5 | Add NaCl | 35.0 g |
| 6 | Stir until all raw materials dissolved |  |
| 7 | Initial pH | 7.13 |
| 8 | Final pH | 7.0-7.2 |
| 9 | 0.5N NaOH used for pH correction |  |
| 10 | 0.5N HCl used for pH correction |  |
| 11 | Add PuW up to 5L |  |

Arginine depletion in formulated BAC2 was performed using UF (ultrafiltration) with cassette of Pall T-series omega 100 kDa, against the replacing buffer.
BAC2 formulation and lyophilization: Arginine depleted BAC2 (prepared by intensive diafiltration of the BAC2 DS) was supplemented with Arg-NaCl solution in ratio of 1:1.67: 85 ml BAC2 + 140ml Arg-NaCl. "Arginine" refers to arginine hydrochloride (Merck, Cat. 1.01544.1000). Solutions were prepared as detailed in Table 4 below. For example, in Sample 2, 3.2 g Arginine were added to 160 ml of NaCl solution. Then 140 ml of this solution were added to 85 ml BAC2 depleted from arginine.

**Table 4:** Arg-NaCl Solutions

| # Formulated Sample | Final Arginine Concentration (g/L) | NaCl Diluent sol. (ml) | Arg (g) | Weight (g) of Solid Samples |
|---|---|---|---|---|
| 1 | 0 | 160 | 0.00 | 2.2103 |
| 2 | 10 | 160 | 3.20 | 2.7419 |
| 3 | 30 | 160 | 9.60 | 3.7768 |
| 4 | 50 | 160 | 16.00 | 4.9636 |
| 5 | 70 | 160 | 22.40 | 6.1397 |

In exemplary procedures, 25 glass bottles (5 for each formulation) were weighed with rubber stoppers and the weighs were recorded to obtain the weights of the empty bottles. Next, 40 ml of diluted BAC2 from each formulation were transfered to 5 bottles. Each bottle was re-capped with rubber stopper loosely (to make sure air can go out of the bottles). All 25 filled bottles were placed randomly on 2 lyophilization trays. Trays were then placed into the middle shelves of the lyophilizer, Christ Martin Epsilon 2-12D LSCplus, (Christ, Osterode am Harz, Germany).
Five replicates of each of the arginine formulation were then lyophilized in Christ Martin Epsilon 2-12D LSCplus, (Christ, Osterode am Harz, Germany) in 100ml bottles (SGD, Type 1, non-siliconized, SGD Pharma Puteaux, France Start the lyophilization cycle (see program in Table 1).
Lyophilization cycle (5 replicates for each formulation, marked "1-5") was carried out for a total of 83 hours with 8 process phases (see Table 1).
At the end of the lyophilization cycle the bottles were closed equilibrated to atmospheric pressure with nitrogen and sealed with a rubber stopper.

After the lyophilization, the bottles were tested for Osmolarity, pH, clottable fibrinogen concentration (by clotting time Clauss method for fibrinogen, see below), cake appearance (visual), foaming (visual generation of excess bubbles) and Factor VIII clotting activity (reconstitution time was tested by the method below).

Reconstitution Method: Materials - In each bottle (vial) the wt. of fibrinogen was about 1 gr and was reconstituted by adding 50 ml of Purified Water (PuW) at room temperature (RT) using Transofix™ 2-way needle, Transofix™ (B. Braun Bethlehem, Pennsylvania, United States) followed by mixing gently by hand to avoid foaming. Reconstitution time was measured for each sample, and average for each formulation was calculated. Reconstitution time was compared to control samples.

Taken together, the following parameters of the samples were eventually evaluated: reconstitution time, fibrinogen recovery (using the Clauss method) and recovery of FVIII (using the FVIII clotting assay). The characteristic of the cake was also evaluated and recorded. The fibrinogen potency Clauss assay and the fibrinogen clottable assay are described in detailed in the Eu pharmacopeia. Specifically:

Fibrinogen by Clauss: the test is a modification of the Eu.Ph. assay 0903/1997, which is based on the Clauss method (Clauss A. Acta Hematol. 1957; 17; 237-246). A calibration curve is prepared with fibrinogen (Enzyme Research lab, South Bend, IN, USA) in the presence of an access of thrombin, and the fibrinogen concentration of the samples are calculated from the calibration curve. Each dilution of the samples is pipetted, and the clotting time is read in clotting machine (Start. Clot. Machine, CLT-101-06 Stago Parsippany, NJ, USA). The results are reported in mg/ml fibrinogen by multiplying the result by the dilution factor of the samples.

FVIII assessment using clotting assay: Test principle - the assay includes the measurement of the clotting time in the presence of cephalin and an activator of a system in which all the factors are present, constant and in excess (supplied by STA©-Deficient FVIII. Parsippany, NJ, USA) except from FVIII which is derived from the sample being tested. The test is performed in clotting machine (Start. Clot. Machine, CLT-101-06 Parsippany, NJ, USA) using STA©-Deficient VIII plasma (Stago, Cat.No 00725). The Factor VIII level is expressed as a percentage of the activity of a normal plasma (where the activity is known) and based on this is calculated in IU/ml.

The results are summarized in Table 5 below.

**Table 5:** Effect of Arginine Concentration (at a fix concertation of protein/fibrinogen) on the Reconstitution Time and Recovery of Fibrinogen (Fib.) and FVIII (five tests for each formula; "#" relates to Formula no.)

| # | Arg mg/ml (final) | NaCl mg/ml (final) | Osmolality** (mOsm/Kg), Measured | pH | Cake Appearance | Average Dissolution Time (min) | Foaming | Fib by Clauss (mg/ml)* | FVIII IU/ml | Fib. per Vial* (g) |
|---|---|---|---|---|---|---|---|---|---|---|
| 1 | 0 | 5.64 | 231 | 7.11 | Non Homogenous, Amorphic | 22:12 | Medium | 20.8 | 4.6 | 1.040 |
| 2 | **7.5** | 5.64 | 290 | 7.2 | Homogenous, Crystalline | 09:08 | Medium | 19.8 | ND | 1.02 |
| 3 | 10 | 5.64 | 311 | 6.95 | Homogenous, Crystalline | 05:17 | Minor/ none | 20.3 | 5.2 | 1.015 |
| 4 | **30** | 5.64 | 468 | 6.81 | Very dense, Crystalline | 08:28 | Minor/ none | 18.8 | 3.6 | 0.940 |
| 5 | 50 | 5.64 | 605 | 6.77 | Dense, Crystalline | 03:49 | Minor/ none | 18.0 | 4.2 | 0.900 |

(continued)

| # | Arg mg/ml (final) | NaCl mg/ml (final) | Osmolality** (mOsm/Kg), Measured | pH | Cake Appearance | Average Dissolution Time (min) | Foaming | Fib by Clauss (mg/ml)* | FVIII IU/ml | Fib. per Vial* (g) |
|---|---|---|---|---|---|---|---|---|---|---|
| 6 | 70 | 5.64 | 769 | 6.69 | Dense, Crystalline | 02:28 | Minor/none | 17.3 | 3.3 | 0.865 |

*L-Arginine monohydrochloride with a molecular wt. of 210.66 was used. The rage of FVIII drug substance is about 3.6-5.3 IU/ml, can be rounded to 3.0-6.0 IU/ml. Thus, **7.5** mg/ml L-Arginine monohydrochloride, per low rage of arginine per high range of FVIII 6 IU/ml = 7.5/210.66/6 X 1000, that is about 6 mmol per IU of FVIII. 30 mg/ml L-Arginine monohydrochloride per the high range of arginine per low range of FVIII: 3 IU/ml = 30/210.66/3 X1000= **47.5** mmol per IU of FVIII; The range of L-Arginine monohydrochloride concentration in the actual solution for lyophilization was in moles: **35-142** (7.5 to 30 in mg). The fibrinogen concentration is less variable because 1 gr is targeted. In terms of total protein, the calculation can be made based on the trending ranges found in BAC2 batches, 60-80% clottable protein. This brings to 25 - 33 mg total protein per ml.

**Osmolarity is measured by osmometer (Advanced Instruments, Inc.Model: 3320; Serial #: 141009848).

As can be seen in Table 5, an optimum of short reconstitution time exists for lyophilized cryo preparation containing a 10-20 mg/ml arginine in the reconstituted sample, compared to samples containing 30 mg/ml arginine, 7.5 mg/ml arginine or no arginine at all. The cakes with arginine at 30 mg/cm$^3$ and more were very dense and compacted thus, consequently, the dissolution time was prolonged. Higher concentration 50 mg/ml and more, although resulting in a very dense cakes were still reconstituted in less than 3 min. However, such a concentration is detrimental for the clinical since, surprisingly, a consistent reduction in fibrinogen and FVIII activity was measured. The reduction in the fibrinogen potency was directly correlated with the increase in the quantities of arginine above 20 mg/ml. Surprisingly, above 7.5 to less than 10 mg/ml the effect on cryo proteins was not significant and the reconstitution time was significantly shorter than with lower concentration of arginine. **Figures 1A-B** summarize the effect of arginine concentration on the reconstitution time, recovery of fibrinogen in viral inactivate lyophilized plasma cryo concentrate. It is noteworthy that that there is a continuous effect of arginine on fibrinogen recovery. The reconstitution behavior is more complex: at first it goes faster and then has no effect. In other words, in terms of reconstitution, a little amount of arginine is sufficient (see the polynomial graph, Figure 1B).

**Example 3: Effect of Fill Volume on the Reconstitution Time End Fibrinogen Recovery**

The lyophilized samples were obtained in small bottles and were then the reconstituted in the same container. The reconstitution time was measured with a timer upon visual inspection of absent of turbidity.

Each bottle was filled with about 1 gr of fibrinogen (as calculated by the total clottable protein, by the assay described by the Eu. Pharmacopeia (Ph. Eur. Monograph for Fibrin Sealant Kit 01/2005:0903). The final fill of one gram per bottle/sample was based on the following calculation: 15 ml of BAC Drug Substance (typically about 70 mg/ ml clottable proteins x 15 ml = 1050 mg clottable protein, see European pharmacopeia monograph for Fibrin Sealant monograph Fibrin Sealant Kit (Ph. European Pharmacopoeia 8.2: 2.7.4) was mixed with either 15 or 25 ml PuW (total of 30 ml or 40 ml filling volume, respectively). The amount of arginine and the sodium chloride per gram of fibrinogen was the same in both filling volumes.

The results are summarized in Table 6.

**Table 6:** Product Attributes results obtained in the Samples Manufactured with 30ml vs. 40ml filling volumes

| Sample | Filling volume* | Dissolution time (min) | Osmolarity (mOsm/kg) | pH | Viscosity (mm$^2$/sec) | Fibrinogen by Clauss (mg/ml)** | Clottable protein (mg/ml)** |
|---|---|---|---|---|---|---|---|
| 1 | 30ml | 6:00 | 330 | 6.89 | 2.6 | 18.6 | 21.2 |
| 2 | 40ml | 2:00 | 329 | 6.92 | 2.6 | 19.8 | 20.5 |

*The samples (with 1 gr fibrinogen) had been lyophilized in small bottles and the reconstitution was then performed in the same container.

**After reconstitution.

The reconstitution time was measured with the timer upon visual inspection, and it was apparent from the results that the reconstitution of the larger volume of 40 ml (i.e. about 40 cm$^3$ lyophilized samples) in 50 ml was shorter than the 30 ml and the fibrinogen activity (measured by Clauss; the Clauss assay and the clottable assay is described in detailed in the Eu pharmacopeia and in Examples 1 and 2) was higher. However, it should be noted that increasing the volume may extent the main drying cycle and may cause variability of the final water content in the 100 ml filled bottles. Therefore, from an industrial point of view replacing the 100 ml bottles with 200 ml would reduce the lyophilizer output and require a larger storage place. Therefore, an amount of 40 ml in a 100 ml bottles seems optimal for an industrial lyophilization process.

**Example 4: The effect of Sodium Chloride**

The aim of this test was to examine sodium chloride concentrations which effect the osmolarity. One gr of fibrinogen, Drug Substance (DS), in 10 replicates was adjusted with NaCl to osmolarity values of $\pm30$ mOsm/kg (270 and 330 mOsm/kg by varying the sodium chloride concentration), which is considered a physiological solution. The increase in the salt concentration intended to test the current paradigm that higher concentration of salt would shorten the reconstitution time of fibrinogen, which is a hydrophobic molecule. Samples of 270 and 330 mOsm/kg were used by varying the sodium chloride concentration. However, the arginine concentration was similar in both osmolarities. The 100 ml-bottle filling value was set to 40 ml.

In these experiments a lyophilized cake was used. The arginine was adjusted to 10 mg/ml, that is, the regular BAC2 sample with 7.5 mg/ml was adjusted to 10 mg/ml before the lyophilization process.

The results show a surprising similar fast reconstitution of both osmolarities as can be noted in Table 7, which provide additional evidence for the lesser or rather no effect of the sodium concentration on the reconstitution time on the one hand, and the need for a meticulous adjustment of the arginine on the other hand. It can be summarized from Examples 3 and 4, that achieving a short reconstitution time (less than 4 min) with various BAC2 DS batches of concentrated cryo requires adjusting the arginine (to 400-600 mg per 1g fibrinogen) and the filling volume is 40 ml in 100 ml bottles. The effect of sodium chloride is negligible. However, it is important to keep the reconstituted concentrated cryo, BAC2, at values above normal saline to prevent the erythrocyte lysis during the long infusion of the reconstituted diluted cryo.

**Table 7:** Effect of Osmolarity (osmolarity adjusted to below or above saline osmolarity) on the Dissolution Time

| Batch | Adjusted NaCl Amount per 1 gr Fibrinogen | Dissolution Time (min) Mean ± SD* | Osmolarity (mOsm/kg) Mean ± SD* |
|---|---|---|---|
| A | 216 mg | 3:06±0.23 | 328.6±2.8 |
| B | 138 mg | 3:40±0:46 | 270.1±3.2 |
| *Mean of 10 samples | | | |

**Example 5: Product Characterization and Batch-To-Batch Variability**

Manufacturing of cryo, its full drying and multiple viral activation process may result in a quite variable process and product in an industrial lyophilization setting. Therefore, there is a need for a robust process that would entertain industrial cryo batches for achieving the same robust product.

During the lyophilization cycle in process development (see developed cycle Table 1), additional 6 full scale batches were produced and fully characterized for the main product attributes. The changes performed in the lyo cycle parameters were not expected to affect the protein composition and other product characteristics, except the residual moisture in the final product. All batches were produced using the same formulation (adjusted with NaCl to meet the physiological osmolarity range and with arginine 300 - 500 mg Arginine / 1gr Fibrinogen), with 40-42 ml filling volume (5% overfilling volume of 40 ml) in 100 ml siliconized Schott bottles. The six batches procedure were fully characterized in order to assess batch-to-batch variability. The obtained results are summarized in Table 8.

**Table 8:** Results Obtained During the Characterization of Six Lyophilized Fibrinogen Batches

| Batch # | Total Protein (mg/ml) | Clottable Fibrinogen (mg/ml) | Clottable Protein (mg/ml) | Fibrinogen by Clauss Method (mg/mL) | Water Content (%) | pH | Osmolality (mOsm/kg) | Viscosity (mm²/sec) | Average Dissolution Time (min) |
|---|---|---|---|---|---|---|---|---|---|
| 1 | 32.2 | 21.7 | 22.7 | 21.77 | 1.09 | 6.90 | 298 | 2.70 | 02:40 |
| 2 | 32.8 | 20.2 | 21.0 | 21.74 | 0.77 | 6.92 | 299 | 2.80 | 02:57 |
| 3 | 34.7 | 22.3 | 22.9 | 22.60 | 1.76 | 6.91 | 322 | 2.87 | 03:07 |
| 4 | 35.2 | 22.8 | 23.0 | 22.00 | 0.86 | 6.92 | 307 | 2.86 | 03:30 |
| 5 | 32.2 | 20.6 | 21.4 | 18.78 | 0.26 | 7.09 | 229 | 2.66 | 02:59 |
| 6 | 32.6 | 21.5 | 23.5 | 20.00 | 0.96 | 6.94 | 290 | 2.90 | 04:00 |

As demonstrated by the experiment described herein, even using different batches, once the arginine concentration was set 400 to 500 mg/g fibrinogen and the fill volume has also been set to 42 (40 ml with 5% overfill) even applying some minor changes in the lyophilized cycle does not significantly change the reconstitution nor the product characteristics.

**Example 6: In Vivo Reconstituted Concentrate Cryo Thrombogenicity**

Bank blood cryo has been used via intravenous infusion, in large quantities, for more than 60 years. Thus, it was important to confirm that the product formulation would not exhibit any thrombogenicity. This was assured by running a in vivo thrombogenicity assay. Aseptically produced samples (dry cake of cryo containing 1-gram fibrinogen) from one batch were fully characterized for its biochemical and physical attributes (Table 9) and then assessed by Wessler stasis model (see Thrombosis Research 132, 2, 280-287, 2013).

**Table 9:** Attribute Results of Samples Characterization from the Batch used for the Assessment of the Product Thrombogenicity

| Parameter | Result |
|---|---|
| Total protein | 30.3 mg/ml |
| Clottable Fibrinogen | 21.7 mg/ml |
| Fibrinogen by Clauss | 21.8 mg/ml |
| FXIII | 0.6 IU/ml |
| Fibronectin | 1.8 mg/ml |
| Glycine | 2.4 mg/ml |
| Arginine Hydrochloride | 10.4 mg/ml |
| Citrate | 0.8 mg/ml |
| Density | 1.02 g/cm$^3$ |
| Water Content | 1.1% |
| pH | 6.9 |
| Osmolality | 298 mOsm/kg |
| Viscosity | 2.7 mm$^2$/sec |

The results in Table 9 provide evidence that all attributes of the formulated lyophilized cryo samples used for the pre-clinical study were well within the desired ranges. The lyophilized cryo demonstrated a very low thrombogenic potential with values that were comparable those measured in standard commercial purified fibrinogen (Haemocomplettan P, CSL Behring, (King of Prussia, Pennsylvania, United States).

**Claims**

1. A composition comprising fibrinogen, Factor VIII, and a positively charged amino acid, wherein the positively charged amino acid comprises arginine, wherein the positively charged amino acid is present at a concentration ranging from above $35 \times 10^{-3}$ mmol per cm$^3$ to below $142 \times 10^{-3}$ mmol per cm$^3$, and wherein the ratio of arginine to total protein ranges from above 0.2 mg per mg protein to below 1 mg of arginine per mg protein.

2. The composition of claim 1, being formulated as a pharmaceutical composition for intravenous administration.

3. The composition of claim 1 or claim 2, being substantially devoid of added hydrophobic amino acid.

4. The composition of any one of claims 1 to 3, wherein:

   (a) the composition comprises more than 5% albumin, by weight of the total proteins; or
   (b) the composition comprises more than 5% up to 25% albumin, by weight of the total proteins; or
   (c) the composition is substantially devoid of added albumin.

5. The composition of any one of claims 1 to 4, being viral-inactivated.

6. The composition of any one of claims 1 to 5, being in the solid form, optionally wherein said solid form is selected from an amorphic form, a crystalline form, a sponge-like form and a powder.

7. The composition of any one of claims 1 to 6, being in the solid form (e.g., in the amorphic or crystalline form, in the sponge-like uniform form, or in the powder form), wherein:

   (a) the composition is **characterized by** dissolution in an aqueous medium within less than 20 min at an atmospheric pressure and 25 °C; or
   (b) the composition is **characterized by** dissolution in an aqueous medium within less than 5 min.

8. The composition any one of claims 1 to 7, wherein the ratio of arginine to fibrinogen ranges from 0.3 to 1.6, respectively, by weight.

9. The composition of any one of claims 1 to 8, further comprising one or more members selected from: factor XIII, fibronectin, and von Willebrand factor, and vitronectin.

10. The composition of any one of claims 1 to 9, wherein the ratio of arginine to total protein ranges from above 0.2 mg per mg protein to 0.4 mg of arginine per mg protein

11. The composition of any one of claims 1 to 10, wherein the composition is a dry pharmaceutical composition capable of being dissolved within less than 6 mins in an aqueous medium, wherein: (i) the ratio of arginine to Factor VIII ranges from above 1.4 to below 8.3 mg/IU; and (ii) the ratio of arginine to fibrinogen ranges from 0.3 to 1.6, respectively, by weight.

12. A method for the preparation a fibrinogen- and Factor VIII-containing product in the solid form, the method comprising the step of drying a solution comprising fibrinogen, Factor VIII, and a positively charged amino acid, wherein the positively charged amino acid comprises arginine, wherein the ratio of the positively charged amino acid to Factor VIII ranges from above 1.4 to below 8.3 mg/IU, and wherein the ratio of arginine to total protein ranges from above 0.2 mg per mg protein to below 1 mg of arginine per mg protein, optionally wherein:

   (a) the solution is viral inactivated; and/or
   (b) the step of drying is carried out by lyophilization; and/or
   (c) the method comprises at least two orthogonal viral inactivation steps of the solution.

13. A method for obtaining a highly-soluble solid composition comprising fibrinogen and Factor VIII, the method comprising adding a positively charged amino acid to a liquid composition comprising said fibrinogen and Factor VIII in a ratio of the positively charged amino acid to Factor VIII ranging from above 1.4 to below 8.3 mg/IU, respectively; and drying said liquid composition so as to obtain the solid composition, wherein the positively charged amino acid comprises arginine, and wherein the ratio of arginine to total protein ranges from above 0.2 mg per mg protein to below 1 mg of arginine per mg protein, optionally wherein the method is devoid of adding a hydrophobic amino acid.

14. A solid composition obtainable by the method of claim 12 or claim 13, optionally wherein the solid composition is in a sponge-like, crystalline or amorphic form.

15. The solid composition of claim 14, wherein:

   (a) the solid composition dissolves in an aqueous medium within less than 5 min; and/or
   (b) the solid composition has a weight ratio of arginine to fibrinogen ranging from 0.3 to 1.6, respectively; and/or
   (c) the solid composition further comprises one or more members selected from: factor XIII, fibronectin, and von Willebrand factor, and vitronectin.

16. A method for obtaining a reconstituted solid fibrinogen in an aqueous medium, the method comprising providing the solid composition of claim 14; and adding an aqueous medium at a volume ranging from above 100% to less than 170%, e.g., 125%, of said solid fibrinogen.

17. A reconstituted solid fibrinogen in an aqueous medium obtainable by the method of claim 16.

18. A kit for obtaining a reconstituted solid fibrinogen, the kit comprising a first container comprising the composition of claim 7; and a second container comprising an aqueous medium.

**Patentansprüche**

1. Zusammensetzung, umfassend Fibrinogen, Faktor VIII und eine positiv geladene Aminosäure, wobei die positiv geladene Aminosäure Arginin umfasst, wobei die positiv geladene Aminosäure in einer Konzentration vorhanden ist, die in einem Bereich von über $35 \times 10^{-3}$ mmol pro $cm^3$ bis unter $142 \times 10^{-3}$ mmol pro $cm^3$ liegt, und wobei das Verhältnis von Arginin zu Gesamtprotein in dem Bereich von über 0,2 mg pro mg Protein bis unter 1 mg Arginin pro mg Protein liegt.

2. Zusammensetzung nach Anspruch 1, die als eine pharmazeutische Zusammensetzung für eine intravenöse Verabreichung formuliert ist.

3. Zusammensetzung nach Anspruch 1 oder 2, die im Wesentlichen frei von zugegebener hydrophober Aminosäure ist.

4. Zusammensetzung nach einem der Ansprüche 1 bis 3, wobei:

   (a) die Zusammensetzung mehr als zu 5 Gew.-% Albumin, bezogen auf die Gesamtproteine, umfasst; oder
   (b) die Zusammensetzung mehr als zu 5 Gew.-% bis zu 25 Gew.-% Albumin, bezogen auf die Gesamtproteine, umfasst; oder
   (c) die Zusammensetzung im Wesentlichen frei von zugegebenem Albumin ist.

5. Zusammensetzung nach einem der Ansprüche 1 bis 4, die virusinaktiviert ist.

6. Zusammensetzung nach einem der Ansprüche 1 bis 5, die in der festen Form ist, optional wobei die feste Form aus einer amorphen Form, einer kristallinen Form, einer schwammartigen Form oder einem Pulver ausgewählt ist.

7. Zusammensetzung nach einem der Ansprüche 1 bis 6, die in der festen Form (z. B. in der amorphen oder kristallinen Form, in der schwammartigen, einheitlichen Form oder in der Pulverform) ist, wobei:

   (a) die Zusammensetzung **gekennzeichnet ist durch** eine Auflösung in einem wässrigen Medium innerhalb von weniger als 20 Minuten bei Atmosphärendruck und 25 °C; oder
   (b) die Zusammensetzung **gekennzeichnet ist durch** die Auflösung in einem wässrigen Medium innerhalb von weniger als 5 Minuten.

8. Zusammensetzung nach einem der Ansprüche 1 bis 7, wobei das Gewichtsverhältnis von Arginin zu Fibrinogen jeweils in dem Bereich von 0,3 Gew. bis 1,6 Gew. liegt.

9. Zusammensetzung nach einem der Ansprüche 1 bis 8, ferner umfassend ein oder mehrere Elemente, die ausgewählt sind aus: Faktor XIII, Fibronectin, von-Willebrand-Faktor und Vitronectin.

10. Zusammensetzung nach einem der Ansprüche 1 bis 9, wobei das Verhältnis von Arginin zu Gesamtprotein in dem Bereich über 0,2 mg pro mg Protein bis 0,4 mg Arginin pro mg Protein liegt.

11. Zusammensetzung nach einem der Ansprüche 1 bis 10, wobei die Zusammensetzung eine trockene pharmazeutische Zusammensetzung ist, die in der Lage ist, sich in einem wässrigen Medium innerhalb von weniger als 6 Minuten aufzulösen, wobei: (i) das Verhältnis von Arginin zu Faktor VIII in dem Bereich über 1,4 bis unter 8,3 mg/IE liegt; und (ii) das Gewichtsverhältnis von Arginin zu Fibrinogen jeweils in dem Bereich von 0,3 Gew. bis 1,6 Gew. liegt.

12. Verfahren für die Herstellung eines Fibrinogen- und Faktor-VIII-haltigen Produkts in der festen Form, das Verfahren umfassend den Schritt eines Trocknens einer Lösung, umfassend Fibrinogen, Faktor VIII und eine positiv geladene Aminosäure, wobei die positiv geladene Aminosäure Arginin umfasst, wobei das Verhältnis der positiv geladenen Aminosäure zu Faktor VIII in dem Bereich von über 1,4 bis unter 8,3 mg/IE liegt und wobei das Verhältnis von Arginin zu Gesamtprotein in dem Bereich von über 0,2 mg pro mg Protein bis unter 1 mg Arginin pro mg Protein liegt, optional wobei:

    (a) die Lösung virusinaktiviert ist; und/oder
    (b) der Trocknungsschritt durch Lyophilisation ausgeführt wird; und/oder
    (c) das Verfahren mindestens zwei orthogonale Virusinaktivierungsschritte der Lösung umfasst.

13. Verfahren zum Erhalten einer hochlöslichen festen Zusammensetzung, umfassend Fibrinogen und Faktor VIII, das Verfahren umfassend das Zugeben einer positiv geladenen Aminosäure zu einer flüssigen Zusammensetzung, umfassend das Fibrinogen und Faktor VIII in einem Verhältnis der positiv geladenen Aminosäure zu Faktor VIII, das jeweils in dem Bereich von über 1,4 bis unter 8,3 mg/IE liegt;

    und Trocknen der flüssigen Zusammensetzung, um die feste Zusammensetzung zu erhalten, wobei die positiv geladene Aminosäure Arginin umfasst und wobei das Verhältnis von Arginin zu Gesamtprotein in dem Bereich von über 0,2 mg pro mg Protein bis unter 1 mg Arginin pro mg Protein liegt, optional, wobei das Verfahren ohne Zugeben einer hydrophoben Aminosäure auskommt.

14. Feste Zusammensetzung, die durch das Verfahren nach Anspruch 12 oder 13 erhältlich ist, optional wobei die feste Zusammensetzung in einer schwammartigen, kristallinen oder amorphen Form vorliegt.

15. Feste Zusammensetzung nach Anspruch 14, wobei:

    (a) die feste Zusammensetzung sich in einem wässrigen Medium innerhalb von weniger als 5 Minuten auflöst; und/oder
    (b) die feste Zusammensetzung ein Gewichtsverhältnis von Arginin zu Fibrinogen aufweist, das jeweils in dem Bereich von 0,3 bis 1,6 liegt; und/oder
    (c) die feste Zusammensetzung ferner ein oder mehrere Elemente umfasst, die ausgewählt sind aus: Faktor XIII, Fibronectin, von-Willebrand-Faktor und Vitronectin.

16. Verfahren zum Erhalten eines rekonstituierten festen Fibrinogens in einem wässrigen Medium, das Verfahren umfassend ein Bereitstellen der festen Zusammensetzung nach Anspruch 14; und das Zugeben eines wässrigen Mediums in einem Volumen, das in dem Bereich von über 100 % bis weniger als 170 %, z. B. 125 %, des genannten festen Fibrinogens liegt.

17. Rekonstituiertes festes Fibrinogen in einem wässrigen Medium, das durch das Verfahren von Anspruch 16 erhältlich ist.

18. Kit zum Erhalten eines rekonstituierten festen Fibrinogens, das Kit umfassend einen ersten Behälter, umfassend die Zusammensetzung nach Anspruch 7; und einen zweiten Behälter, umfassend ein wässriges Medium.

**Revendications**

1. Composition comprenant du fibrinogène, du facteur VIII, et un acide aminé chargé positivement, dans laquelle l'acide aminé chargé positivement comprend de l'arginine, dans laquelle l'acide aminé chargé positivement est présent à une concentration allant de plus de $35 \times 10^{-3}$ mmol par $cm^3$ à moins de $142 \times 10^{-3}$ mmol par $cm^3$, et dans laquelle le rapport de l'arginine aux protéines totales va de plus de 0,2 mg par mg de protéine à moins de 1 mg d'arginine par mg de protéine.

2. Composition selon la revendication 1, étant formulée en tant que composition pharmaceutique pour administration intraveineuse.

3. Composition selon la revendication 1 ou la revendication 2, étant sensiblement dépourvue d'acide aminé hydrophobe ajouté.

4. Composition selon l'une quelconque des revendications 1 à 3, dans laquelle :

    (a) la composition comprend plus de 5 % d'albumine, en poids des protéines totales ; ou

(b) la composition comprend plus de 5 % jusqu'à 25 % d'albumine, en poids des protéines totales ; ou

(c) la composition est sensiblement dépourvue d'albumine ajoutée.

5. Composition selon l'une quelconque des revendications 1 à 4, étant inactivée viralement.

6. Composition selon l'une quelconque des revendications 1 à 5, étant sous la forme solide, facultativement dans laquelle ladite forme solide est choisie parmi une forme amorphe, une forme cristalline, une forme spongieuse et une poudre.

7. Composition selon l'une quelconque des revendications 1 à 6, étant sous la forme solide (par exemple, sous une forme amorphe ou cristalline, sous la forme homogène spongieuse, ou sous la forme de poudre), dans laquelle :

(a) la composition est **caractérisée par** une dissolution dans un milieu aqueux en moins de 20 min à une pression atmosphérique et à 25 °C ; ou

(b) la composition est **caractérisée par** une dissolution dans un milieu aqueux en moins de 5 min.

8. Composition selon l'une quelconque des revendications 1 à 7, dans laquelle le rapport de l'arginine au fibrinogène va de 0,3 à 1,6, respectivement, en poids.

9. Composition selon l'une quelconque des revendications 1 à 8, comprenant en outre un ou plusieurs éléments choisis parmi : facteur XIII, fibronectine et facteur de Von Willebrand, et vitronectine.

10. Composition selon l'une quelconque des revendications 1 à 9, dans laquelle le rapport de l'arginine aux protéines totales va de plus de 0,2 mg par mg de protéine à 0,4 mg d'arginine par mg de protéine.

11. Composition selon l'une quelconque des revendications 1 à 10, dans laquelle la composition est une composition pharmaceutique sèche capable d'être dissoute en moins de 6 min dans un milieu aqueux, dans laquelle : (i) le rapport de l'arginine au facteur VIII va de plus de 1,4 à moins de 8,3 mg/IU ; et (ii) le rapport de l'arginine au fibrinogène va de 0,3 à 1,6, respectivement, en poids.

12. Procédé destiné à la préparation d'un produit contenant du fibrinogène et du facteur VIII sous la forme solide, le procédé comprenant l'étape de séchage d'une solution comprenant du fibrinogène, du facteur VIII, et un acide aminé chargé positivement, dans lequel l'acide aminé chargé positivement comprend de l'arginine, dans lequel le rapport de l'acide aminé chargé positivement au facteur VIII va de plus de 1,4 à moins de 8,3 mg/IU, et dans lequel le rapport de l'arginine aux protéines totales va de plus de 0,2 mg par mg de protéine à moins de 1 mg d'arginine par mg de protéine, facultativement dans lequel :

(a) la solution est inactivée viralement ; et/ou

(b) l'étape de séchage est effectuée par lyophilisation ; et/ou

(c) le procédé comprend au moins deux étapes d'inactivation virale orthogonale de la solution.

13. Procédé permettant d'obtenir une composition solide hautement soluble comprenant du fibrinogène et du facteur VIII, le procédé comprenant l'ajout d'un acide aminé chargé positivement à une composition liquide comprenant ledit fibrinogène et ledit facteur VIII dans un rapport de l'acide aminé chargé positivement au facteur VIII allant de plus de 1,4 à moins de 8,3 mg/IU, respectivement ; et le séchage de ladite composition liquide de façon à obtenir la composition solide,

dans lequel l'acide aminé chargé positivement comprend de l'arginine, et dans lequel le rapport de l'arginine aux protéines totales va de plus de 0,2 mg par mg de protéine à moins de 1 mg d'arginine par mg de protéine, facultativement dans lequel le procédé est dépourvu d'ajout d'un acide aminé hydrophobe.

14. Composition solide pouvant être obtenue par le procédé selon la revendication 12 ou la revendication 13, facultativement dans laquelle la composition solide est sous une forme spongieuse, cristalline ou amorphe.

15. Composition solide selon la revendication 14, dans laquelle :

(a) la composition solide se dissout dans un milieu aqueux en moins de 5 min ; et/ou

(b) la composition solide a un rapport pondéral de l'arginine au fibrinogène allant de 0,3 à 1,6, respectivement ;

et/ou
(c) la composition solide comprend en outre un ou plusieurs éléments choisis parmi : facteur XIII, fibronectine, et facteur de Von Willebrand, et vitronectine.

16. Procédé permettant d'obtenir un fibrinogène solide reconstitué dans un milieu aqueux, le procédé comprenant la fourniture de la composition solide selon la revendication 14 ; et l'ajout d'un milieu aqueux à un volume allant de plus de 100 % à moins de 170 %, par exemple, 125 %, dudit fibrinogène solide.

17. Fibrinogène solide reconstitué dans un milieu aqueux pouvant être obtenu par le procédé selon la revendication 16.

18. Trousse permettant d'obtenir un fibrinogène solide reconstitué, la trousse comprenant un premier récipient comprenant la composition selon la revendication 7 ; et un second récipient comprenant un milieu aqueux.

Fig. 1A

Fig. 1B

## REFERENCES CITED IN THE DESCRIPTION

**Patent documents cited in the description**

- WO 1998055140 A
- US 6121232 A
- US 7727743 B
- US 9943600 B
- US 9814765 B
- US 20130312868 A
- US 5792835 A
- US 7125569 B
- EP 534178 A
- EP 1390485 A
- WO 2013001524 A1
- US 4455300 A
- US 4297344 A
- WO 2008053475 A

**Non-patent literature cited in the description**

- **TZIOMALOS K et al.** *Vasc Health Risk Manag.*, 2009, vol. 5, 843
- **WEINKOVE R** ; **RANGARAJAN S**. *Fibrinogen Transfus Med.*, 2008, vol. 18, 151-7
- **O'SHAUGHNESSY DF** ; **ATTERBURY C** ; **BOLTON MAGGS P et al.** *Br J Haematol.*, 2004, vol. 126, 11-28
- **SØRENSEN B** ; **BEVAN D**. *Br J Haematol.*, 2010, vol. 149, 834-43
- **MASSIMO F.** ; **GIUSEPPE L.** Fibrinogen replacement therapy: a critical review of the literature. *Blood Transfusion*, January 2012, vol. 10 (1), 23-27
- **EISENBERG**. *J. Mol. Biol.*, 1984
- **CLAUSS A**. *Acta Hematol.*, 1957, vol. 17, 237-246
- *Thrombosis Research*, 2013, vol. 132 (2), 280-287